# EUROPEAN PATENT SPECIFICATION

(11) **EP 1 555 982 B1**
(45) Date of publication and mention of the grant of the patent: **30.12.2009**
(21) Application number: 03763767.5
(22) Date of filing: 08.07.2003
(51) Int. Cl.: C11D 17/04, A61K 8/02, A61Q 19/00, A61Q 19/10

(54) **PRODUCTS COMPRISING A DRY APPLICATOR, AN AQUEOUS PHASE AND A LIPID PHASE**
PRODUKTE MIT EINEM TROCKENEN APPLIKATOR,EINER WÄSSRIGEN PHASE UND EINER LIPIDPHASE
PRODUITS COMPRENANT UN APPLICATEUR SEC, UNE PHASE AQUEUSE ET UNE PHASE LIPIDIQUE

(30) Priority: 12.07.2002 EP 02077873; 13.01.2003 EP 03075198
(43) Date of publication of application: 27.07.2005
(73) Proprietor: Johnson & Johnson GmbH, 40474 Düsseldorf (DE)
(72) Inventor: HAUSER, Matthias, 53757 Sankt Augustin (DE); ANSMANN, Achim, 40699 Erkrath (DE); ISSBERNER, Ulrich, 19002 Ambler, PA (US); JACKWERTH, Bettina, 40764 Langenfeld (DE); LEONARD, Mark, Bexley, Kent DA5 1AZ (GB)
(74) Representative: Metten, Karl-Heinz
(86) International application number: PCT/EP2003/007396
(87) International publication number: WO 2004/006869

(56) References cited:
- WO-A1-96/14835
- US-A- 3 334 374
- US-A- 4 390 524
- US-A- 5 947 986
- US-A- 6 153 208
- US-B1- 6 395 301

## Description

### Field of the Invention

This invention concerns products for cleansing and other applications, which products comprise as an applicator a puff (pouf), pad, sponge, cotton ball, swab, brush, glove, mitt or bar, to which a lipid phase and subsequently an aqueous phase have been applied and which products have been dried afterwards. The invention further concerns the manufacture and use of such products.

### Background of the Invention

A plurality of applicators for delivering commodities to a surface have been developed, such applicators being of varied nature, in as well presentation as material selection, e.g. applicators that are resilient or non-resilient, or that are re-usable or disposable. Such applicators have been used to apply to surface ingredients in the form of creams, pastes, gels, liquids, powders and the like. In particular such applicators have been used to apply topical preparations to the skin such as cosmetic, dermatological and the like products. Applicators have been used with a separate product supply or have been impregnated or coated with a measured quantity of product.

One particular type of applicators are wipes which have become an important product category that has found a wide variety of applications for adults and babies. Examples include face or body cleansing wipes, wipes for skin treatment, and skin conditioning wipes. So-called wet wipes have become successful as products particularly suited for these applications.

Developments in the wipe area were focused on the wipe itself, as well as on the wipe material and on the lotions applied thereto. Lotions have been developed which offered skincare benefits in addition to the basic cleansing properties of the wipe.

However, these approaches still leave room for improvement. Firstly, only a small portion of the lotion is released from the wipes during use. Thus a large quantity of the relatively expensive lotion is not delivered to the skin providing no benefit to the consumer and is wasted when the product is discarded after use. This also prevents the use of expensive but more effective ingredients. Secondly, from a formulation point there is an apparent contradiction in the optimization of cleansing performance and skincare benefits in one single lotion, since ingredients which are effective in cleansing usually are not compatible with efficient skin care agents.

Another important factor in cleansing is the fact that a number of soils are water-compatible and therefore more easily removed by water-based formulations, whereas others are lipid-compatible and therefore adequately removed by lipid or oil based formulations. A complete and effective removal of soils therefore requires the presence in or to the applicator of as well water as oil-based components.

This is in particular required in products for personal cleansing and in particular in products used for babies and infants. Inadequate cleaning not only results in personal discomfort but also gives rise to diaper rash and other infection related phenomena. It has been shown that the most effective way of preventing diaper rash is to cleanse the skin thoroughly and to remove the microorganisms that have been identified as causative. The source of these microorganisms is often the fecal deposits that can remain on a baby's skin while wearing the diaper. Because fecal deposits consist of both water-soluble and oil-soluble matter, however, complete removal of fecal deposits from the diaper area requires both water-based and oil-based cleansing agents.

US-4,987,632 discloses a substantially dry-to-the-touch wiping article for use in cleaning soiled surfaces wherein moisture barriers cover the surface of the sheet. WO 99/13861 and US-6,153,208 disclose substantially dry personal cleansing articles wherein the substrate comprises multiple layers. US-6,280,757 concerns cleansing articles that are dry comprising a substrate having apertures of certain size and frequency.

In US 5,947,986 a hygienic applicator having moist and/or dry swab end sections which may be covered by wrappers to prevent contamination is disclosed. The moist end sections can be pre-moistened with a desired liquid such as a soap solution, cleaning solution, baby wipe solution, baby oil, mineral oil or a medicant. The dry end sections can also be moistened with a preferred liquid and dried. With the hygienic applicator of US 5,947,986 a means is provided to effectively clean the ear canal, the external ear, or other external skin surface in both wet and dry environmental conditions.

According to US 3,334,374 a dry applicator pad is provided containing a supply of liquid agent in the form of a multitude of individual loose dry pressure-rupturable capsules having a diameter of less than 1.000 µm. The liquid agent can be readily released in controlled amounts by pressure on the pad during use. Accordingly, the pads described in US 3,334,374 are dry in storage but contain within them a readily release supply of the liquid agent.

US 6,395,301 B1 is about an anhydrous hydrophobic cosmetic composition in the form of a compact powder which comprises a particulate phase comprising 20 to 35% by weight of hydrophilic pulverulent compounds. This compacted composition can also contain fatty substances chosen from oils or waxes of mineral, animal or plant origin, silicone origin, fluoro oils, fatty acid esters and/or mixtures thereof. The composition according to US 6,395,301 B1 can be used either in dry form or with water. In the latter case an applicator can be employed which has been moistened beforehand.

WO 96/14835 A1 discloses wipes which comprise a carrier substrate such as tissue paper web which has been treated with a water-in-lipid emulsion wherein the internal water phase contains a high level of water. This high internal phase inverse emulsion ruptures when subjected to low shear during use, e.g., wiping of the skin or other surfaces, so as to release the internal water phase. WO 96/14835 A1 provides products for perianal cleaning that have consistent levels of moistening solution, have adequate temporary wet strengths so as to be flushable, have an adequate, consistent moisture level to provide effective cleaning, and remain essentially dry until use for cleaning purposes.

In US 6,153,208 a dispersible, single use personal care cleansing and conditioning article is disclosed which comprises a water-insoluble substrate and at least one lathering surfactant, wherein said article is substantially dry prior to use. The water-insoluble substrate has to have at least a first portion which is wet extensible and at least a second portion which is less wet extensible than said first portion. Furthermore, selected portions of said first portion have to be jointed to said second portion in a manner which is sufficient to inhibit wet extension of said first portion in the plane of said first portion. Additionally, said selected portions of said first portion have to be bonded to said second portion to provide a continuous bonded region which defines a plurality of discrete unbonded regions. In such a manner the cleansing and conditioning articles of US 6,153,208 provide affected cleansing using low, and hence less irritating levels of surfactant while providing superior conditioning benefits. Whereas traditional applicator products have been based on the applicator material having one phase, the products of this invention concerns the application of two distinctly different phases onto or into an applicator. Both phases differ in terms of physical properties and may be applied on various parts or portions of the applicator. This approach allows a combined optimal cleansing performance and superior skincare properties.

### Summary of the Invention

This invention relates to products that comprise an applicator, other than a porous or absorbent sheet, for transferring ingredients to surfaces and in particular to the skin, according to the features of claim 1.

Dry refers to the situation where the water content is very low, i.e. lower than 1 % and essentially dry means that the product contains limited amounts of water, e.g. less than 10 % of the total weight of the product, preferably less than 8 %, more preferably less than 5 %, still more preferably less than 2 %.

A product as defined herein comprises an applicator to which a lipid phase has been applied and to which subsequently an aqueous phase is applied, whereafter the product is dried.

Preferably, the lipid phase is solid or semi-solid at ambient temperature and more preferably is present at the surface or at the surface portion of one or several sides of the applicator.

In a further aspect, this invention relates to products as defined herein wherein the lipid phase is waxy.

The lipid phase preferably has a low water content, in particular lower than 10%. The lipid phase preferably contains an active ingredient.

The products of this invention are dry or essentially dry. In particular embodiments of the invention dry refers to the situation where the water content is relatively low, i.e. lower than 1 % and essentially dry means that the product contains limited amounts of water, e.g. less than 10 % of the total weight of the product, preferably less than 8 %, more preferably less than 5 %, still more preferably less than 2 %.

In particular said applicator is any three-dimensional substrate capable of transferring ingredients to a surface, in particular the user's skin, namely puffs, pads, sponges, brushes, cotton balls, gloves, mitts or cotton tipped swabs.

The applicators may be made of a variety of materials which are structured such that they are capable of holding and/or absorbing a lipid and an aqueous phase. The materials of which the applicators are made therefore may be porous or absorbent in nature. The materials in particular are polymeric and may be both from natural and non-natural origin.

In a further aspect there is provided a method of manufacturing a product as described herein which is in accordance with the features of claim 19. Said method comprises applying to the applicator a lipid phase and subsequently an aqueous phase. In a preferred method of manufacturing, said applicator is first coated with a lipid phase and subsequently sprayed or impregnated with an aqueous phase.

In still another aspect the invention provides the use of a product as described herein as a combined cleanser and applicator of active substances.

### Detailed Description of the Invention

The applicator in the products according to this invention can be resilient or non-resilient. The applicator can be used as such or can have a suitable handle. It can take any tridimensional form that is suited for application to flat surfaces including the skin. The applicators can be of different size and take a variety of forms, e.g. flat or not, geometrically shaped or not, round which includes cylindrical, ellipsoidal, spherical and the like shapes, or angular shaped such as square or rectangular, which includes cubic or bar shapes, also with rounded edges or combinations of these shapes. One or more of the outer sides of the applicator may be made of different materials having different properties. For example one side may be soft while another side is rougher. The latter side can be abrasive, it can be used for rubbing or scouring. The applicators can be hard, soft, semi-soft, resilient or not, squeezable or not.

One type of embodiments are puffs (poufs), pads, brushes, gloves, mitts, swabs or cotton balls.

Another type of embodiments are sponges. Sponges comprise sponges as such, foams and felts, composed of synthetic and/or natural materials.

For convenience of use, the applicators may have a suitable handle. Embodiments of such applicators have a pad, puff or sponge portion that preferably is resilient and a finger grip portion. One type of such applicators are those having a generally T-shaped configuration. Examples of such applicators comprise resilient discs with a small upstanding handle element.

The applicators can be made of materials which are capable of holding, adsorbing or absorbing a lipid and an aqueous phase. Preferably, the applicator material is structured such that it is porous or absorbent in nature. The latter can be due to the chemical structure of the applicator materials or their physical arrangement or both. Examples of particular physical arrangements are porous structures, or cellular or microcellular structures.

The applicators can be made of one type of material or from different materials that can be arranged in different manners along the applicator. Small portions of one or more materials of different or equal size may be incorporated into a matrix of the same or another material. Or the applicators can be multilayered such as a stack of layers or concentric layers or they can be of one type of material. Applicator parts, either or not of different materials can be linked together by gluing, sewing, stitching or any other technique known in the art.

The materials of which the applicators are made in particular are polymeric and may be both from natural and non-natural origin. There can be one or more polymeric materials that may be cross-linked or not. Optionally other non-polymeric materials such as binders, fillers, dyes and the like, may additionally be present.

The materials can be more or less inert or they can be decomposable, in particular they can be biodegradable. The materials may also be flushable. As used herein, by 'flushable' is meant that the material will pass through at least 3 meters of waste pipe in two toilet flushes.

Examples of polymeric materials of which the applicators are composed are non-natural polymers such as polyethylene, polypropylene, PET, polyamide, polyvinyl alcohol, polyurethane, and the like, and natural or natural-derived polymers such as cellulose, wood pulp and the like and mixtures of such synthetic and natural fibres or materials.

Where the applicator is in the form of a puff (pouf) it can be composed of spongy or resin foamy materials, optionally wrapped in a suitable mono- or multilayered material, which can be made of a closed or an apertured material layer or film. In other embodiments the puff is made of one or more layers of material that can be bound or glued together in the core of the puff.

If layered materials are used, these materials in themselves may be mono or multi-layered, woven or non-woven. They can be made of one or of several materials. Particularly preferred layered materials are made of non-woven materials that have a web structure of fibrous or filamentous nature, in which the fibres or filaments are distributed randomly or with a certain degree of orientation, the former being obtainable by air-laying or certain wet-laying processes, the latter in other wet-laying or in carding processes. The fibres or filaments can be natural, for example wood pulp, wool cotton, linen and the like, or synthetic, for example polyvinyls, polyesters, polyolefins, polyamides and the like.

Multi-layered sheet materials have two or more layers of the same of different materials, woven or non-woven, or layers obtained by different techniques. One embodiment is a material composed of three layers, e.g. polyethylene /pulp/polyethylene or viscose/polypropylene/viscose.

One type of non-woven materials is paper based, which are made almost exclusively of cellulose-based fibres. Where high wet strength or firmness of the layered material is desired, binding materials can be added. Softness can be increased by adding additives. In anothertype of non-wovens the web is made mainly of staple fibre, e.g. based on cotton, wool, linen and the like.

Usually, non-woven materials for use in the applicatore of the invention are made of cellulose fibres, synthetic fibres such as polyester or polyprolpylene, or mixtures thereof. Webs of increased strength can be obtained by using the so-called spunlace or hydro-entanglement technique, which does not require binding material.

One type of non-woven materials are made of a mixture of pulp and staple fibre and are available with binding materials, in particular those mentioned above, or without binding materials. In the latter instance the non-woven is preferably made by the hydro-entanglement procedure.

### The Two Phases

In the products according to this invention, the applicator material is contacted with a lipid and an aqueous phase. In some embodiments the applicator is contacted with a third phase which may be a polymeric phase.

The product is dried after the aqueous phase has been applied.

The applicator material is first treated with the lipid phase and subsequently with the aqueous phase, after which the thus obtained product is dried.

Also included is the possibility to apply multiple aqueous and multiple lipid phases and to introduce several drying steps. In each step it is possible that the phase is applied to only a portion of the applicator, or to one side of the applicator, or two or more sides. Any combination of such applications of the phases are deemed within the ambit of the present invention.

The products of the invention are dry or essentially dry. Dry refers to the situation where the water content is very low, i.e. lower than 1 %. As used herein essentially dry means that the product contains limited amounts of water, e.g. less than 10 % of the total weight of the product, preferably less than 8 %, more preferably less than 5 %, still more preferably less than 2 %. It more generally means that after manufacture, no water or aqueous-based lotion is added to the applicator. As used herein a % is w/w to the total weight of the applicator with all materials incorporated therein of thereon.

The aqueous phase may be applied to the whole applicator, i.e. continuously, or to parts of the applicator, i.e. discontinuously. One phase may be applied continuously while the other, namely the lipid phase, is applied discontinuously. They can be applied at the surface or in the internal of the applicator. If applied at the surface, one or both phases can be present at one side or at several sides of the applicator, or one phase may be present at one side while the other phase is present at the other side of the applicator.

In the instance where a phase or both phases are applied discontinuously, they are present in or at certain areas, in particular in or at one or more areas of the applicator. In that instance, the phase or phases may be present as one or more forms or shapes. For example they can be present as dots or spots, lines or stripes, as geometrical figures such as squares, rectangles, circles and the like, as symbols such as letters, text, logos, figures and the like, or as trademark signs, or any other such forms, or a combination thereof. The forms or shapes may be present over the entirety of the applicator or grouped in one or more areas, for example in a corner.

In a particular embodiment, the lipid phase is applied on one or on several sides of the applicator in the form of stripes, dots or other forms covering the entire surface or only a part of the surface of the applicator. The aqueous phase is applied to the applicator either on the entire surface of the applicator or on certain areas. This is done in a second step after the application of the lipid phase.

First the lipid phase and subsequently the aqueous phase are applied to the applicator.

Different parts of the applicator may contain different aqueous and/or lipid phases. For example the applicator may at one side contain one lipid phase and at another side another lipid phase. Or in other embodiments, the applicator at one side may contain the lipid phase while at the other side contains aqueous phase.

Or the applicator may be composed of two or more parts that are linked together; each part having been treated with a different lipid phase. This may result for example in applicator that at one portion has cleansing capacity and at an other portion has caring capacity.

Where the applicator is in the form of a puff, a pad or a sponge it may be coated with lipid phase, which preferably is solid, or the puff may have a lipid phase, which may be liquid, semi-liquid or solid, deposed at the inner portion of the applicator. If deposed at the inner portion, the lipid phase may be distributed homogeneously, meaning that is distributed over the whole inside in more or less equal quantities, or inhomogeneously.

Where the applicator is in the form of a sponge, it can be wrapped into a sheet of material to which a lipid phase may be applied. Furthermore, the bar or sponge material itself may contain the same or different lipid phase(s). The lipid phase at the outside preferably is solid while at the inside can be solid, semi-solid or liquid. The lipid phase at the inner portion of the applicator may have been deposited or the applicator may have been impregnated with lipid phase material in liquid form, which afterwards may solidify. This type of applicators further contains the aqueous phase which may be at the surface layer or at the inside.

Where the applicator is in the form of a puff, the lipid phase may have been applied in a powdery form.

Where the applicator is in the form of a sponge it may be made of a decomposable material such as a biodegradable material. For example it can be made of dissolvable cellulose, which can be mixed with lipid phase when the cellulose is still in a liquid state during the production process.

### The lipid phase

The lipid phase that is applied to the applicator is such or formulated such that it is insoluble or essentially insoluble in the aqueous phase. However, in some embodiments the two phases may be mixable or soluble into each other to a limited extend. The lipid and aqueous phase should be such or formulated such that once on the sheet and for the time prior to usage of the sheet product by the consumer they do not form one phase or a continuous phase.

The lipid phase is hydrophobic and is composed of materials that are generally insoluble in water such as oils or fats, or waxes. The lipid phase can be semi-solid or solid at ambient temperature. The lipid phase can be semi-solid, the latter term having the standard meaning used in the art. It can be amorphous, semi-crystalline or crystalline, or it can take the form of a cream or waxy composition.

Semi-solidness can occur when the lipid phase is in a transition stage between solid state and liquid state such as in a melting process, but can also be due to increased viscosity of the material that makes up the lipid phase. Semi-solidness is present in materials having a waxy, creamy, pasty, gelly or similar consistency. Semi-solidness in particular occurs with materials that have no sharp melting point, i.e. materials that have a melting range. It is also present in glass-like materials, e.g. in polymers that occur as in a glass-like state.

The lipid phase has a melting point or a melting range above room temperature, i.e. above or equal to 25 °C, for example in the range of 25 to 100°C, in particular in the range of 30 to 75°C, more in particular of 30 to 45°C, preferably in the range of 32 and 40°C. More preferably the melting temperature or melting range is above human body temperature. Most preferably the melting temperature or melting range approximates or is equal to human body temperature.

In some embodiments of this invention the lipid phase may have a relatively higher melting point or range. The melting point or range may for example be higher than body temperature, e.g. higher than 40 °C, or higher than 45 °C. Upon application of such products, a more intense interaction between the two phases may be required or the application of higher temperatures, to promote the interaction. In the latter instance the consumer may, for example, be required to contact the product first with hot water and then to apply it. In the former instance the aqueous phase may contain agents that promote a stronger interaction with the lipid phase.

As used herein the term 'melting range' refers to a temperature range that starts from the temperature at which a substance or composition loses its solid consistency up to the temperature where it becomes completely liquid. A melting range is considered to be within a defined temperature range when it overlaps with that defined temperature range, or should be considered to be above a specified temperature when the range is above said temperature.

As used herein 'ambient temperature' refers to a temperature that is in the range of about 20 to about 25 °C.

The lipid phase can change to another state after application to the applicator or when being applied to the applicator during storage, or upon usage by the consumer. The lipid phase may be applied to the applicator as a liquid where after it becomes semi-solid or solid. Or the lipid phase may become semi-solid or liquid during usage by the consumer.This change of state may be induced by physical factors such as temperature or pressure but may also be induced by chemical factors such as particular components that cause a polymerization reaction or by a photochemical reaction.

In certain embodiments, the lipid phase may be applied as two separate phases which become mixed during application on to the applicator, whereupon certain components in each phase become mixed and start to interact, e.g. in a polymerization reaction thus changing the state of the lipid phase from liquid to semi-solid or solid.

Particularly preferred are the compositions of the lipid phase which are solid at room temperature and which have a penetration value of 0.2 - 4 mm (measured with: Petrotester PNR 10, Mikrokonus, 5 sec., temp 20 °C).

The water content of the lipid phase is low, in particular less than 10 %, preferably less than 6 %, more preferably less than 3 %. In a particular embodiment the lipid phase is water free, and will be such that it is not decomposed by the aqueous phase. As used herein, 'water free' means that the phase is composed of materials of low water content to which no water has been added.

The lipid phase may comprise one or more components selected from oils or fats, or waxes. It may further contain other components. As used herein oils or fats refer to the same type of materials, oils being liquid at ambient temperature and fats being solid or semi solid at ambient temperature. The lipid phase may also comprise mixtures of waxes and fats and/or oils.

In a preferred embodiment, the lipid phase is a wax-based composition, wherein the term 'wax' is as specified hereinafter.

In particular embodiments, multiple lipid phases, i.e. lipid phases of different composition, may be applied to the applicator. For example one type of lipid phase is applied to one side of the applicator while another type is applied to the other. Each of these lipid phases may or may not contain one or more of the ingredients mentioned hereinafter, for example one or more ingredients selected from the active ingredients, the dyes, emulsifiers, and other ingredients mentioned hereinafter. In case of various dyes, multi-colored patterns may exist, for example, each lipid phase may have a different color or may be uncolored.

The different lipid phases may be applied differently at each side of the applicator. For example one side may completely be covered while at another side the lipid phase is applied in a pattern, e.g. as stripes.

### Oils and fats

The lipid phase may contain oils, fats or mixtures of fats with oils and/or with oily components. The resulting mixture of which the lipid phase is composed should preferably be selected such that the melting point or melting range of the lipid phase is as mentioned above, i.e. is above ambient temperature, more in particular is in the range of 32 °C to 40 °C.

Oils or fats which can be used in the lipid phase comprise natural oils or fats, or natural oil or fat derivatives, in particular of vegetable origin. Examples are almond oil, soybean oil, sunflower oil, safflower oil, corn oil, kernel oil, canola oil, borage oil, evening primrose oil, grapeseed oil, wheat germ oil, avocado oil, jojoba oil, sesame oil, walnut oil, linseed oil, palm oil, olive oil, macadamia oil, castor oil, rapeseed oil, peanut oil, coconut oil, turnip seed oil, and the hardened derivatives thereof. The latter are obtained by hydrogenation of fats or oils. Preferred are hardened oils or fats from vegetal origin, e.g. hardened castor oil, peanut oil, soya oil, turnip seed oil, cotton seed oil, sunflower oil, palm oil, kernel oil, linseed oil, almond oil, corn oil, olive oil, sesame oil, cocoa butter, shea butter and coconut oil.

Said hardened fats or oils have the additional advantage of increasing the consistency of the lipid phase compositions.

The lipid phase may further comprise fatty components isolated from these natural oils, i.e. pure triglycerides or mixtures thereof, or the latter components having been prepared chemically. These so-called trigycerides (or triacyl glycerines) are esters of glycerines with fatty acids or fatty acid mixtures, for example so called technical mixtures obtained by hydrolysis from fractions of oils or fats, or by fractioning fatty acid mixtures after hydrolysis. The triglycerides may also be obtained chemically by synthesis.

The fatty acids in said triglycerides may be saturated or unsaturated, straight or branch chained, substituted or unsubstituted. In a first embodiment the lipid phase of the product of the invention comprises triglycerides that are those glycerines esters derived from fatty acids, either saturated or unsaturated, having from 12 to 24, preferably from 16 to 20 carbon atoms. Preferred such fatty acids are, for example, palmitic, palmic, oleic, lauric, myristic, stearic, hydroxystearic, behenic acid, or mixtures thereof. Within this group the triglycerides derived from saturated fatty acids are of particular interest.

Of specific interest are glyceryl tristearate, also referred to as stearin, glycerine tribehenate, glycerine tripalmitate, glycerine trilaurate, glycerine trioleate, glycerine trimyristate.

The lipid phase may also contain mono- or diglycerides, optionally in a mixture with the fats and oils mentioned herein, in particular with triglycerides. The mono- or diglycerides for use in the lipid phase are derived from saturated or unsaturated, linear or branch chained, substituted or unsubstituted fatty acids or fatty acid mixtures. Also in this instance the melting point or melting range of the lipid phase preferably is as mentioned above, in particular is above ambient temperature, more in particular is in the range of 32 °C to 40 °C. Particular mono- or diglycerides are mono- or di-C₁₂₋₂₄ fatty acid glycerides, specifically mono- or di-C₁₆₋₂₀ fatty acid glycerides, for example glyceryl monostearate, glyceryl distearate. Mixtures of mono-, di- and, optionally, triglycerides can be derived from fractions of fatty acids. An example of such mixture for use as a component of the lipid phase is a mixture of C₁₂₋₁₈ mono-, di- and triglycerides.

In a preferred embodiment according to the present invention the lipid phase contains one or more fatty acid glycerides selected from the mono-, di- or triesters from glycerine, or a mixture thereof.

The glycerides can be present in various amounts, it is typically present in an amount of up to 60% or in certain embodiments up to 70 %, or up to 80 % (w/w), relative to the total quantity of the lipid phase.

In other embodiments, in particular those containing dialkyl(ene)ethers or -carbonates, dicarboxylic acids or hydroxy fatty alcohols, the amount of said fatty ester glycerides will be less than 50 % and more preferably less than 40 % (w/w), relative to the total quantity of the lipid phase.

In a particular aspect of this invention there provided products as specified herein wherein the lipid phase consists essentially of one or more fatty acid glycerides selected from the mono-, di- or triesters from glycerine, or mixtures thereof. The glyceride can be present in various amounts, e.g. the amounts mentioned hereinabove or hereinafter.

Mixed esters as well as mixtures of mono-, di- and triglycerides are of particular interest because of their low propensity to crystallize and their capacity to improve the consistency of the formulation making up the lipid phase.

The lipid phase may also comprise alkyl esters of fatty acids, wherein the alkyl group has from 1 to 30 carbon atoms, preferably from 12 to 24 carbon atoms. The fatty acids in said alkyl esters in particular are C₁₂₋₃₀ fatty acids, more in particular C₁₂₋₂₀ fatty acids. The alkyl groups in said esters preferably are derived from fatty alcohols as well as of mixtures thereof, which, for example, are obtained by high pressure hydrogenation of technical mixtures of the methyl esters derived from fats or oils.

Preferred are the alkyl esters of C₁₆₋₂₄ fatty acids, more preferably from C₁₆₋₁₈ fatty acids, and C₁₋₃₀ fatty alcohols, preferably C₈₋₂₄ fatty alcohols, more preferably C₁₂₋₂₀ fatty alcohols.

Of particular interest in this regard are, e.g. stearyl stearate, palmityl stearate, stearyl behenate, cetyl stearate, cetyl behenate, cetyl palmitate, cetearyl behenate, behenyl behenate, stearyl heptanoate, stearyl octanoate, myristyl myristate, myristyl isostearate, myristyl oleate, cetyl isostearate, cetyl oleate, stearyl isostearate, stearyl oleate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl isostearate, behenyl oleate, erucyl isostearate.

Of further interest are esters of linear C₆-C₂₂-fatty acids with branched alcohols, in particular 2-ethylhexanol, esters of branched C₆-C₂₂-fatty acids with linear alcohols, esters of C₁₈-C₃₈-alkylhydroxycarbonic acids with linear or branched C₆-C₂₂-fatty fatty alcohols, esters of linear and/or branched fatty acids with poly-alcohols (e.g. propylene glycol, dimerdiol or trimertriol) and/or Guerbet alcohols, as well as esters of C₆-C₂₂-fatty alcohols and/or Guerbet alcohols with aromatic carbonic acids, in particular benzoic acid, esters of C₂-C₁₂-dicarbonic acids with linear or branched C₁-C₂₂ -alcohols (e.g. dioctyl malate) or C₂-C₁₀-polyoles having 2 to 6 hydroxy groups.

Preferred fats comprise the triglycerides, in particular those derived from fatty acids having from about 12 to about 24 carbon atoms, in particular those having from about 12 to about 20 carbon atoms, more in particular those having from about 16 to about 20 carbon atoms. These fatty acids may be unsaturated or, which is preferred, saturated.

Particularly preferred are glycerides derived from oleic, stearic, myristic or lauric acid, or from fatty acid mixtures derived from natural oils such as coco-acids. Examples of preferred fats are cocoglycerides, glyceryl stearate, glyceryl laurate, and the like.

Further preferred fats comprise hydrogenated natural oils such as hydrogenated castor oil, hydrogenated palm oil and the like.

The lipid phase may also comprise oily components, i.e. non water-mixable components that are liquid at 20 °C. These can be e.g. glycerides, hydrocarbons, silicon oils, ester oils and the like, as well as mixtures thereof. The total quantity of these oily components in the total composition of the lipid phase will be such that the lipid phase is solid at room temperature, or that it has a melting point or range that is as specified hereinabove. The oily components will typically be present in quantities of less than 40 % (w/w), in particular less than 20 % (w/w), or further in particular 1- 15 % (w/w), more in particular from 2 - 10 % (w/w) relative to the total weight of the lipid phase.

The oily components can be any of the oils mentioned hereinabove as 'oils and fats', more in particular the mono-, di- and triglycerides mentioned hereinabove, that are liquid at 20 °C. The oily components can further be fatty acids and fatty alcohols, described in this specification, that are liquid at 20 °C.

Further oily components which can be used in the lipid phase comprise silicone oils, mineral and paraffin oils and synthetic oils, either aliphatic or aromatic, as well as mixtures thereof. Examples of such oils are squalane, squalene, isohexadecane, isoeicosane, polydecene, and also oils of the group of dialkylcyclohexanes.

The lipid phase may further contain silicone oils, volatile or not, such as, for example, cyclic silicones, dialkyl- or alkylarylsiloxanes, e.g., cyclomethicone, dimethyl polysiloxane (dimethicone) and methylphenyl polysiloxane, as well as the alkoxylated and quaternized derivatives thereof. Appropriate non-volatile silicone oils are e.g. longer chain polyalkylsiloxanes and polyalkylarylsiloxanes, and also polyethersiloxane-copolymers.

The total amount of fats or oils, or of mixtures of fats and oils and/or oily components in the lipid phase in particular is at least 50 %, preferably at least 70 %, more preferably at least 90 %, w/w of the total amount of components making up the lipid phase.

In a particular aspect of this invention there are provided products as specified herein wherein the lipid phase essentially consists of fats or oils, or of mixtures of fats and oils and/or oily components, in particular those specified in this specification. The fats, oils and oily components can be present in various amounts, e.g. the amounts mentioned hereinabove or hereinafter.

### Waxes

The lipid phase may be composed of or may comprise waxes. As used herein, the term 'wax' refers to oil soluble materials that have a waxy consistency and have a melting point or range of above or equal to 25 °C. Waxes are materials that have a solid to semi-solid (creamy) consistency, are crystalline or not, being of relative low viscosity a little above their liquefying point. Waxes can be composed of one or more components, synthetic as well as natural, and can in principle be composed of or comprise any oil soluble material having a waxy consistency.

The lipid phase may be composed of or may comprise waxes that are synthetic or natural waxes, as well as other oil soluble materials that have a waxy consistency. Waxes also encompass materials such as oils or fats of natural or synthetic origin, and waxy components such as higher alkanols (in particular fatty alcohols), higher alkanediols (in particular hydroxy fatty alcohols), carboxylic acids (in particular fatty acids), dialkyl(ene)ethers, dialkyl(ene) carbonates, dicarboxylic acids and the like components.

Natural waxes comprise waxes from vegetal origin, such as purcelline, shea butter, cocoa butter, Japan wax, esparto gras wax, cork wax, Guaruma wax, rice shoot wax, Ouricury wax, montan wax, sunflower wax, ceresine wax, sugar cane wax, carnauba wax, candelilla wax, lanolin, fruit-derived waxes, such as orange wax, lemon wax, grapefruit wax and bayberry wax, and the like, and of animal origin such as beeswax, woolwax, spermateci and bear fat, shellac wax, and the like. Natural waxes further comprise mineral waxes such as ceresine and ozokerite waxes. Synthetic waxes comprise petroleum-based waxes such as paraffin, vaseline, petrolatum, micro wax. Further synthetic waxes are polyalkylene and polyethyleneglycol waxes, e.g. polyethylene wax; waxes based on chlorinated naphtalenes such as 'Halowax', synthetic hydrocarbon waxes, and the like, including mixtures thereof. Further waxes are chemically modified waxes, in particular hardened or hydrogenated waxes such as, for example, Montan-ester waxes, Sasol waxes and hydrogenated jojoba waxes. Preferred among these natural waxes are waxes from vegetal origin.

Other wax components can be certain fats (including mono-, di- and triglycerides and fatty acid alkylesters), fatty alcohols, fatty acids, including substituted fatty acids (in particular hydroxy substituted fatty acids, for example, 12-hydroxystearic acid), dialkyl(ene)ethers, dialkyl(ene) carbonates, dicarboxylic acids (in particular the C₁₆-C₄₀-dialkylesters of dicarboxylic acids, e.g. the C₁₆-C₄₀-alkyl stearates, C₁₈-C₃₈-alkylhydroxystearyl stearates or C₂₀-C₄₀-alkyl erucates) and hydroxy fatty alcohols that comply with the definition of 'wax' as outlined herein. Any of these components may contain homologous components that are liquid, as long as the total composition making up the lipid phase has a waxy consistency. For example, waxy fats may contain oils, waxy fatty alcohols may contain liquid fatty alcohols, etc., in such amount that the total composition has a waxy consistency and in particular has the melting point or melting range specified above.

Still further wax components are selected from the group of aromatic carbonic acids, tricarboxylic acids, or from the group of lactides of long-chained hydroxycarbonic acids. Myristyl lactate is particularly attractive for use on applicators for skin treatment, because of its binding capacity to the skin.

Further wax components that can be used are C₃₀-C₅₀-alkyl bees wax; tri-C₁₆-C₄₀-alkyl citrates, e.g. tristearyl citrate, triisostearyl citrate, trilauryl citrate; ethyleneglycol difatty acid esters, in particular the ethylene glycol di-C₁₂-C₃₀-fatty acid esters, e.g.ethyleneglycol dipalmitate, ethyleneglycol distearate, ethyleneglycol di(12-hydroxystearate). As further useful wax components there can be mentioned siliconewaxes.

The lipid phase may also comprise mixtures of waxes and fats and/or oils.

The total amount of waxes in the lipid phase in particular is at least 50 %, preferably at least 70 %, more preferably at least 90 %, w/w of the total amount of components making up the lipid phase.

In a particular aspect of this invention there are provided products as specified herein wherein the lipid phase essentially consists of one or more waxes selected from the waxes mentioned herein, including mixtures thereof. The waxes can be present in various amounts, e.g. the amounts mentioned hereinabove or hereinafter.

### Fatty alcohols

In a second embodiment the lipid phase of the product of the invention comprises C₁₂-C₂₄-fatty alcohols, e.g. as derived from natural fats, oils or waxes such as, for example, myristyl alcohol, 1-pentadecanol, cetyl alcohol, 1-heptadecanol, stearyl alcohol, 1-nonadecanol, arachidyl alcohol, 1-heneicosanol, behenyl alcohol, brassidyl alcohol, lignoceryl alcohol, ceryl alcohol or myricyl alcohol as well as Guerbet alcohols. Preferred for use in the present invention are saturated, straight or branch chained fatty alcohols. However also unsaturated, straight or branch chained alcohols can be used, optionally in a mixture with saturated alcohols. Preferably the alcohols will be selected such that the melting point of the mixture is as referred to hereinabove and more in particular is in the range of 32 to 40 °C.

Mixtures of fatty alcohols can evidently also be used, including fatty alcohol fractions obtained from the reduction of the corresponding fatty acid fractions derived from naturally occuring oils or fats such as, for example, almond oil, soybean oil, sunflower oil, safflower oil, corn oil, canola oil, borage oil, evening primrose oil, grapeseed oil, wheat germ oil, avocado oil, jojoba oil, sesame oil, walnut oil, linseed oil, palm oil, olive oil, castor oil, macadamia oil, rapeseed oil, peanut oil, coconut oil, and turnip seed oil.

Synthetic alcohols can also be used such as, for example, the linear fatty alcohols of an even number of carbon atoms resulting from the Ziegler-synthesis (Alfole^{®}) or the partially branched alcohols resulting from the Oxo synthesis (Dobanole^{®}).

A preferred embodiment according to the present invention is that wherein the lipid phase contains at least one C₁₄-C₁₈-fatty alcohol. Also preferred is a lipid phase with at least one C₁₆/C₁₈-Guerbet alcohol.

The use of fatty alcohols advantageously results in the lipid phase having a drier, i.e. less greasy, skin feel, compared to components such as triglycerides.

The total amount of fatty alcohols in the lipid phase may vary and depends on the desired properties of the lipid phase. In a number of instances it is desirable to have a relative higher quantity of fatty alcohols in the composition, in particular said alcohols will be present in an amount of 50 %, preferably at least 70 %, more preferably at least 90 %, (w/w) of the total amount of components making up the lipid phase. In other instances, relatively lower amounts are desired, the total amount of the fatty alcohols present in the lipid phase may be in the range of 1- 40 %, preferably of 1 - 30 % (w/w), more preferably of 1 - 20 % (w/w), still more preferably from 1 -10 % (w/w).

In a particular aspect of this invention there provided products as specified herein wherein the lipid phase essentially consists of one or more fatty alcohols, in particular those specified in this patent specification, including mixtures thereof. The fatty alcohols can be present in various amounts, e.g. the amounts mentioned hereinabove or hereinafter.

### Fatty acids

In a third embodiment the lipid phase of the product of the invention contains fatty acids, e.g. C₁₄-C₄₀-fatty acids, including mixtures thereof. Of particular interest are the C₁₆-C₃₀-fatty acids. These comprise, for example, myristic-, pentadecanoic-, palmitic-, margaric-, stearic-, nonadecanoic-, arachic-, behenic-, lignoceric-, cerotic-, melissic-, erucaic-, elaeostearic-, oleic-, lonolenic-, lauric acid as well as substituted fatty acids, e.g. hydroxy-substituted fatty acids such as, for example, 12-hydroxystearic acid, and the amides or monoethanolamides of these fatty acids.

The total amount of the C₁₄-C₄₀-fatty acids present in the lipid phase, relative to the total weight amount of the lipid phase, may be in the range of 1 - 30 % (w/w), preferably of 1 - 20 % (w/w), more preferably from 1 -10 % (w/w).

In a particular aspect of this invention there are provided products as specified herein wherein the lipid phase essentially consists of one or more fatty acids, in particular those specified in this patent specification, including mixtures thereof. The fatty acids can be present in varying amounts, e.g. the amounts mentioned hereinabove or hereinafter.

### Dialkyl(ene)ethers or carbonates, dicarboxylic acids or hydroxy fatty alcohols

In a fourth embodiment the lipid phase of the product of the invention contains dialkyl(ene) ethers, dialkyl(ene) carbonates, dicarboxylic acids or hydroxy fatty alcohols, or mixtures thereof, which ethers, carbonates, acids or alcohols in particular those described hereinafter.

In a particular aspect of this invention there are provided products as specified herein wherein the lipid phase essentially consists of one or more dialkyl(ene) ethers or - carbonates, dicarboxylic acids or hydroxy fatty alcohols, including mixtures thereof. The dialkyl(ene) ethers or -carbonates, dicarboxylic acids or hydroxy fatty alcohols can be present in various amounts, e.g. the amounts mentioned hereinabove or hereinafter.

The addition of dialkyl(ene) ethers or -carbonates, dicarboxylic acids or hydroxy fatty alcohols, including mixtures thereof to the composition of the lipid phase allows to optimize the properties of the lipid phase, in particular its sensoric properties, i.e. the products as well as the skin after the products have been applied have a less greasier feel and also a less dry skin-feel, while having excellent skin caring properties.

### Dialkyl(ene) ethers

The dialkyl(ene) ethers are symmetric or asymmetric, straight or branch chained, saturated or unsaturated. Preferred are waxy, saturated C₁₆-C₃₀-dialkylethers, in particular C₁₆-C₂₄-dialkylethers. More preferred are C₁₆-C₂₀-dialkylethers, and particularly preferred are distearylethers and dibehenylethers. Dialkylethers of shorter chain length can also be used such as, for example, di-n-octylether, di-(2-ethylhexyl)-ether, laurylmethylether or octylbutylether, didodecylether. When using the latter components the complete composition of the lipid phase preferably is solid or semi-solid having the desired melting point as specified herein.

These ethers can be obtained from the appropriate fatty alcohols in the presence of an acid catalyst following art-known procedures. Typical examples are the products that are obtained by the etherification of capron alcohol, capryl alcohol, 2-ethylhexyl alcohol, caprin alcohol, lauryl alcohol, myristyl alcohol, cetyl alcohol, palmoleyl alcohol, stearyl alcohol, isostearyl alcohol, elaidyl alcohol, petroselinyl alcohol, linolyl alcohol, linolenyl alcohol, oleyl alcohol, ricinus alcohol, elaeostearyl alcohol, arachidyl alcohol, gadoleylalcohol, behenyl alcohol, erucyl alcohol and brassidyl alcohol, Guerbet alcohols, as well as mixtures thereof, which, for example, are obtained by high pressyre hydrogenation of technical mixtures of the methyl esters derived from fats or oils.

Of particular interest are the dialkyl(ene) ethers that are solid at 25 °C.

### Dialkyl(ene) carbonates

The dialkyl(ene) carbonates are symmetric or asymmetric, straight or branch chained, saturated or unsaturated. Preferred dialkyl(ene) carbonates are waxy, linear or branch chained, saturated or unsaturated C₁₄-C₃₀-dialkyl(ene) carbonates. More preferred are C₁₆-C₂₄-dialkyl carbonates and amongst these the saturated linear C₁₆-C₂₂-dialkyl carbonates. Particularly preferred is distearyl carbonate. Also liquid dialkyl(ene) carbonates, such as, for example, dihexyl-, dioctyl-, di-(2-ethylhexyl)- or dioleylcarbonate, can be used. When using the latter components the complete composition is solid or semi-solid, having the desired melting point as specified herein.

These dialkyl(ene) carbonates can be obtained by re-esterification of dimethyl- or diethylcarbonates with the corresponding hydroxy compounds following art-known procedures. Typical examples of dialkyl(ene) carbonates are re-esterification products of dimethyl- and/or diethylcarbonate with capron alcohol, capryl alcohol, 2-ethylhexyl alcohol, caprin alcohol, lauryl alcohol, myristyl alcohol, cetyl alcohol, palmoleyl alcohol, stearyl alcohol, isostearyl alcohol, elaidyl alcohol, petroselinyl alcohol, linolyl alcohol, linolenyl alcohol, oleyl alcohol, ricinus alcohol, elaeostearyl alcohol, arachidyl alcohol, gadoleyl alcohol, behenyl alcohol, erucyl alcohol and brassidyl alcohol, Guerbet alcohols, as well as technical mixtures thereof, that can be obtained by hydratation of methyl esters derived from suitable oils or fats or oil or fat fractions.

Of particular interest are those dialkyl(ene) carbonates that are solid at 25 °C.

### Dicarboxylic acids

Dicarboxylic acids that can be used are, for example, C₉-C₃₄-dicarbonic acids. Of particular interest are those dicarboxylic acids that are solid at 25 °C.

### Hydroxy fatty alcohols

The hydroxy fatty alcohols for use in the lipid phase are saturated or unsaturated, straight chain or branched. Preferred are C₁₂-C₃₀-hydroxy fatty alcohols, at which the position of the hydroxy-substituent depends upon the synthesis route and the starting materials that have been used. Included are, for example, 1,10-decanediol, 1,2-hexadecanediol, 12-hydroxystearyl alcohol or hydroxy-Guerbet alcohols. Preferred are those hydroxy fatty alcohols that are solid at 25 °C, although liquid analogs can also be used. When using the latter components, the complete composition preferably is solid or semi-solid, having the desired melting point as specified herein. Particularly preferred is 12-hydroxystearyl alcohol.

The total amount of one or more of the dialkyl ethers, dialkyl carbonates, dicarbonic acids and the hydroxyalcohols present in the lipid phase, relative to the total weight amount of the lipid phase, may be in the range of 1 - 30 % (w/w), preferably of 1- 20 % (w/w) more preferably from 1 -10 % (w/w).

### Further components

The compositions of the lipid phase may contain further components, which may be of waxy nature or otherwise. The use of these further components allows to influence the sensorical properties as well as the stability of the compositions, in particular after application to applicator material and more in particular when in contact with the aqueous phase. The other components may also be added to influence consistency, feel and appearance. These components will generally be insoluble or poorly soluble in water. Water-soluble components can also be included, typically in combination with a solubilizing or emulsifying agent and some water.

Examples of further components are superfatting agents, thickeners, polymers, active ingredients, film forming agents, UV-filters, anti-oxidants, hydrotropic agents, preservatives, insect repellents, self-tanning agents, solubilizers, perfume oils, dyestuffs and the like.

These further components may be present in the lipid phase in amounts which are in the range of from 0 - 30 %, in particular from 0.1 - 20 %, more in particular from 1 - 15 %, further in particular from 5 -10 %.

Substances that can be used as superfatting agents are, for example, lanolin or lanolin derivatives such as lanolin alcohols, lanolin acids, polyethoxylated or acylated lanolin, or other lanolin derivatives; phospholipids such as lecithin or lecithin derivatives such as polyethoxylated or acylated lecithin or other lecithin derivatives; polyol fatty acid esters, monoglycerides and fatty acid alkanolamides.

Appropriate cationic polymers are for example cationic cellulose derivatives , e.g. quaternized hydroxyethyl cellulose (commercialized under the trade name Polymer JR 400^{®} by Amerchol), cationic starches, copolymers of diallylammonium salts and acrylamides, quaternized vinylpyrrolidone/vinylimidazole-polymers (for example Luviquat^{®} of BASF), condensation products of polyglycols and amines, quaternized collagen polypeptides, such as, for example, lauryldimonium hydroxypropyl hydrolyzed collagen (Lamequat^{®}L/Grünau), quaternized wheat polypeptides, polyethylene imines, cationic silicone polymers, e.g. amodimethicone, copolymers of adipinic acid and dimethylaminohydroxypropyldiethylenetriamine (Cartaretine^{®}/Sandoz), copolymers of acryl acid with dimethyldiallylammonium-chloride (Merquat^{®} 550/Chemviron), polyaminopolyamides, cationic chitine derivatives such as, for example, quaternized chitosans, optionally dispersed in microcristalline form, condensation products derived from dihalogenalkylenes, such as, for example dibromobutane with bis-dialkylamines, e.g. bis-dimethylamino-1,3-propane, cationic guar-gum derivatives, such as, for example, Jaguar^{®} CBS, Jaguar^{®} C-17, Jaguar^{®} C-16 from Celanese, quaternized ammonium salt-polymers, e.g. Mirapol^{®} A-15, Mirapol^{®} AD-1, Mirapol^{®} AZ-1 from Miranol.

Anionic, zwitterionic, amphoteric and nonionic polymers that can be used are, for example, vinylacetate/crotonic acid-copolymers, vinylpyrrolidone/vinylacrylate-copolymers, vinylacetate/butylmaleate/ isobornylacrylate-copolymers, methylvinylether/maleic acid anhydride-copolymers and their esters, which are not cross-linked and with polyoles linked polyacrylacids which are cross-linked, acrylamidopropyl trimethylammonium chloride/ acrylate-copolymers, octylacrylamide/methylmethacrylate/tert.butylaminoethylmethacrylate/2-hydroxypropylmethacrylate-copolymers, polyvinylpyrrolidone, vinylpyrrolidone/vinylacetate-copolymers, vinylpyrrolidone/ dimethylaminoethylmethacrylate/vinyl caprolactam-terpolymers as well as optionally derivatized cellulose ethers and silicones.

As further consistency agents there can be used small amounts of alkalimetal or alkaline earth metal as well as aluminium salts of C₁₂-C₂₄-fatty acids or C₁₂-C₂₄-hydroxyfatty acids, preferred being calcium-, magnesium-, aluminium- and in particular zinc stearates.

The lipid phase may further contain suitable anti-oxidants such as, for example, sulfites, e.g. sodium sulfite, tocopherol or derivatives s thereof, ascorbic acid or derivatives s thereof, citric acid, propyl gallate, chitosan glycolate, cysteine, N-acetyl cysteine plus zinc sulfates, thiosulfates, e.g. sodium thiosulfates, polyphenoles and the like.

The lipid phase may further contain powders or powdered ingredients or mixtures thereof such as talcum, Bolus alba, myristyl alcohol, cetyl alcohol, cetylstearyl alcohol, calcium or magnesium stearate, magnesium lauryl sulfate, starch or derivatives thereof.

The lipid phase may further contain disintegrating agents, which are agents that cause a disintegration of the physical integrity of the lipid phase. The disintegration may be in parts or on the whole of the lipid phase. The disintegrating agents may be mixed or dissolved into parts or the whole of the lipid phase. The disintegrating agents may be mixed continuously in the lipid phase or discontinuously, e.g. at the top side of the lipid phase, e.g. where the lipid phase is applied as a layer, at the top of that layer or in the top portion of that layer.

Suitable disintegrating agents are agents that are subject to physical or chemical interactions either by auto-interaction or by interaction between two agents. This results in a physical or chemical interaction with the lipid phase. One type of disintegrating agents are those that release a gas e.g. by decomposition or by chemical reaction between two components. An example of a disintegrating agent is a solid mixture of a bicarbonate and an acid such as sodium or potassium bicarbonate with a suitable organic acid, e.g. citric acid. Upon contact with water, e.g. upon contact with the aqueous phase, the disintegrating components will interact and liberate carbon dioxide which physically alters the lipid phase. Such physical alteration may cause the lipid phase to become homogeneously distributed on the applicator. This may positively influence the interaction between the aqueous and lipid phases, which in turn may, for example, have a positive effect on the transfer to the skin of materials, e.g. active ingredients, in these phases.

The lipid phase may further contain components that are subject to a polymerization reaction either during or after application on the applicator material. Examples of such components are oligomers that during or after application on the applicator continue to polymerize with monomers or other oligomers. Other examples are agents that cause netting or co-polymerisation. There can also be agents that inhibit polymerization for a specific period of time. Alternatively there can be agents that accelerate polymerization e.g. under influence of external factors such as heat, light or pressure.

In one type of embodiments, the lipid phase contains monomers or oligomers that can be caused to polymerize or co-polymerize under the influence of an external factor, an example of the latter being light. The lipid phase is applied to the applicator and during the application process the lipid phase is subjected to light radiation whereupon polymerization occurs. Alternatively, the lipid phase may be subjected to light radiation after it having been applied to the applicator.

The lipid phase may further contain dyes that upon usage of the product change color due to a change of temperature or pressure. This will give the consumer a level of comfort and trust that the product delivers the lipid phase to the skin, or in case of a lipid phase containing active ingredients that the latter are delivered onto the skin.

The lipid phase may further contain dye-precursors, i.e. agents that become dyed upon influence of physical or chemical factors. In particular embodiments the lipid phase may contain dye-precursors which react with certain agents that are present in the aqueous phase so as to form a dye. Similarly, the dye-precursors may be present in the aqueous phase and become transferred into dyes upon interaction with certain chemicals incorporated into the lipid phase.

The lipid phase can also be formulated to or into beads. Particular such beads are polymeric beads wherein the lipid phase is entrapped in whatever form. The terms 'beads' or 'polymeric beads' are meant to comprise any form of discrete, free-flowing powders, beads or capsules which envelope, coat or contain a lipid phase in a mono- or polymeric matrix or capsule. These terms also encompass powders, beads or capsules wherein the mono- or polymeric matrix itself is a lipid phase. These terms are also meant to include porous beads or 'microsponges' and 'microcapsules', the latter being beads of smaller size. The beads may be coated with a suitable coating material that protects the interior of the bead or controls the release of the lipid phase entrapped therein. The coating on the bead itself may contain a lipid phase. In the latter instance, the coating is layed on an inert core or on a core containing lipid phase and/or other ingredients.

Formulation of a lipid phase in beads may be done for protecting the lipid phase from external factors that may impact its integrity. However, it is mostly done for allowing controlled release of the lipid phase.

A particular type of beads are small beads or capsules, having an average diameter which is in the micrometer range, although the average diameter can be as small as even 200 nm.

This type of capsules can be liposome-based, made for example of phospholipids such as lecithin, phosphatidyl ethanolamine, phosphatidyl serine, phosphatidic acid and the like. This type of capsules also can be made of starch, cellulose, porous gelatin and the like.

The capsules or beads can also be relatively larger, having average sizes in the mm or 0.1 mm range. This type of capsules or beads can be made of materials such as agar, glycolic acid polymers, and further components such as water, mineral oils, glycerin. They may contain further ingredients such as preservatives, dye(s), and the like.

Another type of beads or microcapsules are microsponges. These are materials sized from about 5 to about 300 µm (average diameter) having a large inner surface. These are obtained by polymerization of particular monomers. Lipid phase material can be entrapped therein either during this polymerization process or afterwards. Microsponge-based carriers may be used to protect the lipid phase entrapped therein or for controlled release purposes.

The capsules may optionally contain one or more suitable disintegrating agents, in particular those mentioned in this specification. Upon contact with the appropriate external factor, the disintegrating agents will cause the capsules to break open thus allowing release of the lipid phase entrapped therein.

The capsules can be incorporated into the aqueous phase or into another lipid phase, or in both. They can also be applied to the applicator prior to the introduction of the lipid and aqueous phase. They can even be introduced during the manufacturing process of the applicator itself.

Release of the lipid phase from the beads or capsules can be the result of the rupture of the coating or from the matrix. This may be the result of physical factors such as pressure, strain or by shearing forces upon use of the applicator product, e.g. by rubbing the product to the skin or to a surface. Release of the lipid phase may be due to the semi-permeable or porous nature of the bead or its coating or due to external factors such as contact with liquid media that cause the lipid phase to become extracted, or to dissolve or disintegrate the bead or its coating, or by temperature effects. The capsules can also be disintegrated under influence of certain chemicals, in particular by disintegrating agents incorporated into the capsules. Particular embodiments of the latter are capsules containing suitable amounts of bicarbonate and an organic acid which, upon contact with water, e.g. upon contact with the aqueous phase when using the applicator product, cause the capsules to disintegrate.

The beads or capsules can be made according to methodologies generally known in the art, for example by emulsion polymerisation.

The beads or capsules may be applied to any portion of the applicator but preferably they are concentrated at the surface or in the upper surface portion of the applicator. This allows maximal transfer of the lipid phase to the skin or to the surface to which the product is applied.

The beads or capsules can be applied to the applicator in dry form by dusting, sifting, spraying and the like methods. They can also be pointed or roll-coated in the form of a suitable liquid or paste. They can also be mixed with a suitable liquid, which can be a solvent that is inert towards the beads, or water, or the aqueous phase, and sprayed onto the applicator.

In preferred embodiments, the composition of the lipid phase has a melting point or melting range of above 25 °C, preferably in the range of 30 to 45 °C, more preferably in the range of 32 to 40 °C.

The water content of the preferred compositions of the lipid phase is low, e.g. lower than 10 %, preferably lower than 6 %, more preferably lower than 3 % w/w relative to the total weight of the lipid phase. In particular, the preferred compositions will be water free.

In said first embodiment the lipid phase comprises one or more fatty acid mono-, di- or triglycerides or natural oils comprising mono-, di- or triglycerides as well as the hydrogenated derivatives of said natural oils, containing from 12 to 24 carbon atoms. A particular example of a hydrogenated derivative of a natural oil is hydrogenated castor oil. The lipid phase in particular comprises a C₁₆₋₂₀ fatty acid mono-, di- or triglyceride. In particularly preferred embodiments I, the lipid phase comprises one or more C₁₆₋₂₀ fatty acid triglycerides. Particular examples of such triglycerides are glyceryl stearate, glyceryl oleate, glyceryl laurate, glyceryl myristate, cocoglycerides, hydrogenated palm glycerides.

The total amount of fatty acid mono-, di- or triglyceride(s) containing from 12 to 24 carbon atoms in the lipid phase according to the first embodiment of the invention (embodiments I) is at least 50 %, preferably at least 70 %, more preferably at least 90 %, w/w of the total amount of components making up the lipid phase. In certain embodiments the total amount of triglyceride(s) in the lipid phase of the embodiment I may be at least 70 %, still more preferably at least 90 %, w/w of the total amount of components making up the lipid phase.

In said second embodiment of the invention the lipid phase contains C₁₂-C₂₄-fatty alcohols, e.g. as derived from natural fats, oils or waxes such as, for examples, myristyl alcohol, 1-pentadecanol, cetyl alcohol, 1-heptadecanol, stearyl alcohol, lauryl alcohol, oleyl alcohol, palmityl alcohol, cetearyl alcohol, 1-nonadecanol, arachidyl alcohol, 1-heneicosanol, behenyl alcohol, brassidyl alcohol, lignoceryl alcohol, ceryl alcohol or myricyl alcohol as well as Guerbet alcohols.

Of particular interest for use in the invention are C₁₄-C₁₈-fatty alcohols as well as C₁₆-C₁₈-Guerbet alcohols.

The total amount of one or more of the C₁₂-C₅₀-fatty alcohols present in the lipid phase, relative to the total weight amount of the lipid phase, may be in the range of 1 - 30 % (w/w), preferably of 1 - 20 % (w/w) more preferably from 1 -10 % (w/w).

In said fourth embodiment of the invention the lipid phase is a waxy composition comprising at least one oil or wax component selected from dialkyl(ene) ethers, dialkyl(ene) carbonates, dicarboxylic acids or hydroxy fatty alcohols or mixtures thereof.

Particular dialkyl(ene) ethers, dialkyl(ene) carbonates, dicarboxylic acids or hydroxy fatty alcohols for use in the lipid phase of said fourth embodiment are those mentioned hereinabove.

The said preferred or particularly preferred waxy compositions preferably liquefy above 25 °C and/or have a water content of less than 10 %, preferably less than 6 %, more preferably less than 3 %. In particular said preferred or further preferred waxy compositions are water-free, and will be such that they are not decomposed by the aqueous phase. As used herein, water-free generally means that the phase is composed of materials of low water content to which no water has been added.

The lipid phase having a composition according to said fourth embodiment can contain the same further ingredients as those described in relation to the lipid phase, in particular further waxy lipid components or oils.

The lipid phase having a composition according to said fourth embodiment may also contain liquid dialkyl(ene) ethers, dialkyl(ene) carbonates, dicarbonic acids or hydroxy fatty alcohols, however preferably in such amounts that the melting point or range of the total composition of the lipid phase does not exceed 25 °C, and more preferably is within the temperature ranges mentioned above.

In particularly preferred embodiments, the products of this invention have a lipid phase containing:
(a) from 1 - 50 % (w/w), in particular from 1 - 30 %, of an oily or waxy component selected from C₁₄-C₃₀-dialkyl ethers, C₁₄-C₃₀-dialkyl carbonates, C₄-C₃₄-dicarbonic acids or C₁₂-C₃₀-hydroxyfatty alcohols or mixtures thereof
(b) 0 - 5 % (w/w), in particular from 0.1- 5 % (w/w), of at least one active ingredient
(c) 0 - 10 % (w/w), in particular from 1 - 10 % (w/w), of at least one oil
(d) 0 - 10 % (w/w), and in particular from 0.1-10 % (w/w), of at least one emulsifier
(e) 0 - 90 % (w/w), and in particular from 5 - 90 % (w/w) of further waxy components
(f) 0 - 5 % (w/w), and in particular 0 - 3 % (w/w), water.

### Application of the lipid phase

The lipid phase may be applied to the applicator in various ways. Preferably the lipid phase is applied at the surface or at the surface portion of the applicator, on one or on several sides.

The lipid phase can be applied evenly or non-evenly to the applicator, non-evenly meaning that the distribution of the amount of the lipid phase varies over the area of the applicator, i.e. some areas of the applicator can have greater or lesser amounts of the lipid phase. Preferably the lipid phase is evenly applied to the area of the applicator.

The lipid phase is applied discontinuously to one or several sides of the applicator.

The lipid phase is applied in a discontinuous pattern, to one or several sides of the applicator. To this purpose the lipid phase is applied in a predetermined, controlled manner to specific areas of the applicator. A discontinuous pattern is one in which the lipid phase has been applied to distinct regions separated by regions of the applicator which are free of the lipid phase. The lipid phase in that instance is applied to defined parts or regions of the applicator which may take a variety of forms. The lipid phase may in particular be applied as described above more generally for the application of both phases. Particular forms in which the lipid phase may be applied are, e.g. stripes, dots or spots, geometric configurations, either of regular or irregular shape, for example circles, ellipses, squares, rectangles and the like, logos, text, letters or any other non-continuous pattern, including the patterns described hereinabove more generally for the application of the lipid and aqueous phase.

Discontinuous patterns also comprise essentially networks of larger patterns of the lipid phase. In a preferred embodiment, the lipid phase is present as discrete stripes which can be disposed discontinuously, i.e. interrupted, or preferably continuous over the whole surface of the applicator. The stripes may also form a pattern of discrete segments which collectively comprise a stripe or they may have a repetitive pattern such as a sinusoidal shape or wave-like and the like pattern. If waving stripes are selected, preferably the stripes are in phase, so that parallelism is maintained and each stripe remains equally spaced from the adjacent stripes.

The stripes are preferably oriented in the machine direction, for ease of manufacture.

In certain embodiments more than one lipid phase may be applied to one or several sides of the applicator. For example one lipid phase may be applied on part of the surface of one side of the applicator, whereas another lipid phase is applied on the other side only partly, either with the same or another pattern than the other lipid phase. Particular such embodiments are those having two different lipid phases on the same side e.g. in parallel stripes or other patterns with the same or different colors.

In particular embodiments, not more than half of the surface of the applicator, either on one side or, which is preferred, on several sides is carrying or covered by the lipid phase. In a preferred embodiment, the lipid phase is present at the surface on several sides, covering not more than 50 % of the applicator's surface, in particular covering not more than 35 % or not more than 25 % of the surface. In a particularly preferred embodiment, the lipid phase is present as stripes, in particular as parallel stripes running in parallel with the side of the applicator, covering not more than half or, more in particular 25 % of the surface. In another particularly preferred embodiment, the lipid phase is present as dots, equally spread over the entire surface of the applicator, covering not more than 50 % of the surface.

Some embodiments have more or less regularly shaped dots, other embodiments will have circle-shaped dots, others have ellipsoids, while still others have mixed patterns, e.g. combinations of circles and ellipsoids, of regularly shaped dots and circles and the like.

In case of stripes, the width thereof preferably is between 1 to 10 mm, more preferably between 3 to 7 mm. In case of dots, round shapes are preferred, e.g. circles or ellipsoids, with an average diameter between 1 to 10 mm, more preferably between 3 to 7 mm. There can be stripes with different widths on one product, and there can be dots of different size on one product. An example of an embodiment of the latter is an applicator with circles of a certain size and ellipses of a different size, or of circles with different sizes.

The lipid phase may be colorless or colored, i.e. mono- or multi-colored. Multi-colored patterns are obtained by applying several lipid phases that have been dyed differently. A colored lipid phase will alert the user of the fact that the applicator is covered by a special material that contains an active ingredient or it may also make the product aesthetically attractive.

In another embodiment the applicator itself is colored, either at several sides or only at one side, over the complete surface or only at parts. If the color is present only at parts of the applicator it preferably will take the shapes and forms described in connection with the patterns that the lipid phase may take. In other embodiments only the space between the surface portions at which the lipid phase is applied is colored thus leaving the areas of the lipid phase uncolored. In this way, the patterns of the lipid phase will appear as uncolored patterns.

A preferred pattern for coloring the applicator is in stripes. Examples of such embodiments are those wherein the colored stripes or the area between the colored stripes are covered with lipid phase. In the former instance the lipid phase stripes are colored, in the latter they are uncolored.

The lipid phase, which itself can be colored or uncolored, may be applied to the colored applicator in a number of different ways. In case of applicators having a completely colored surface, the lipid phase can be applied over the whole surface thus resulting in a different or altered color, e.g. a more pale color where the lipid phase is white or opaque. The lipid phase can also be applied in certain patterns, thus resulting in multicolored products or where the lipid phase is white or opaque in products with mono-colored patterns. Also in this instance, the preferred pattern is in stripes.

In still a further embodiment, the applicator is colored in certain patterns and the lipid phase is applied on these patterns or part of these patterns. Also in this instance the lipid phase may be colored or uncolored, i.e. white, opaque or transparent. In case the lipid phase is white or opaque its thickness may be selected such that the color of the underlying section of the applicator is visible thus giving the consumer the impression that a lipid phase containing a particular ingredient is present.

The lipid phase is typically applied in an amount of from about 3 to about 40 g/m², preferably from about 10 to about 20 g/m², either on one side or, preferably, on several sides of the applicator. Or, alternatively, the lipid phase is applied in an amount of about 0.06 g to about 0.8 g per gram of substrate, preferably from about 0.20 g to about 0.40 g per gram of dry substrate.

The lipid phase can be applied to the applicator by any method that can be used to contact or impregnate a molten lipid material to or in a applicator. Where the latter is solid or semi-solid at room temperature, it is liquefied by melting or dissolving into a suitable solvent which is evaporated afterwards.

The lipid phase can also be applied by any method that allows coating of the lipid material onto the surface of the applicator. As used herein the term 'coating' refers to printing, covering, overlaying, finishing, spraying, extruding, laminating or any other method of applying the phase to the surface of the applicator.

A particular coating technique is extrusion wherein the composition is forced through tubes in contact with the applicator while the applicator passes across the tubes. A preferred technique comprises contacting the applicators with a heated head equipped with a slit blade, i.e. a blade having cut-out areas, wherefrom the lipid phase, in molten state, is extruded. Another preferred coating technique involves the so-called hot melt process which comprises spraying the liquefied lipid phase from a heated spraying head or nozzle. Another application technique involves spraying or drippling the composition on a rotating surface such as calender roll that then transfers the composition to the surface of the substrate.

Still another technique is based on traditional printing technologies which comprise, for example, screen printing, roller printing and gravure printing. In general, printing comprises techniques wherein a rotating surface is provided with elevations (by engraving, embossing or similar techniques) and the elevations are contacted with the liquefied lipid phase, e.g. by running it through a bath with liquefied phase one, and thus printed on the applicator. Another technique to apply the lipid phase is by using a screen printing procedure where the molten lipid phase is introduced into a rotating roll and squeezed through a metal screen which covers the roll. This leads, depending on the design of the screen, to a defined pattern on the applicator like stripes, dots, squares, circles and the like, or even logos and text.

A further technique to apply the lipid phase onto the applicator is by roller-ball application which comprises contacting a ball which is in direct contact with the applicator, with lipid phase in liquid state and transferring it through a rolling movement onto the applicator. Depending on the desired pattern of the lipid phase on the applicator, there can be several of such roller-ball applicators mounted next to one another, or after one another. They may contain the same or different lipid phases.

The lipid phase may also be applied by high-pressure coating. In one type of execution of this procedure the lipid phase is applied via extrusion through appropriate nozzles, under high pressure. Specially shaped nozzles may be used resulting in particular patterns. For example there can be nozzles that result in circles, stars, squares, or other geometric shapes or even irregularly shaped patterns.

The lipid phase may also be applied by a combination of these application techniques.

The lipid phase may also be applied to the applicator as particles or as powder. In one type of embodiments the lipid phase is applied as beads or small capsules, e.g. by drippling or screen printing. After application the particles are caused to melt thereby forming small dots in or on the applicator.

The lipid phase is applied in its molten form.

The lipid phase may be applied in liquid form while being in admixture with water, which can be colored or uncolored and which is removed after application to result in a dry or essentially dry product. 'In liquid form' in this context means that the lipid phase is liquid in itself or is liquefied by heating, e.g. by heating in the water in which it is applied. The lipid phase is kept liquid all along the process. In the instance of a solid lipid phase, it is only allowed to solidify after removal of the water that has been added. In one embodiment, the lipid phase is mixed with hot water whereupon the lipid/water mixture is applied to the sheet. The water is subsequently evaporated which may be accomplished by a variety of means, e.g. by simply allowing the water to evaporate, by passing the applicator over one or more heated members, thus forcing the water to evaporate, by applying dry air, either heated or not, by applying reduced pressure.

In the execution where the water is colored, it will diffuse into the applicator and after its evaporation leave the applicator colored. The lipid phase that has been applied in this type of embodiment may be uncolored, in which case it will appear as white or lighter areas. Or the lipid phase may be colored which will result in a multi-colored product. In another execution, the lipid phase in this process is colored and uncolored water is used resulting in products wherein the lipid phase areas are colored and the areas and the other areas are uncolored. The thus obtained products are subsequently be treated with aqueous phase which may be colored or not, resulting in products with even more color combinations.

In one type of embodiments, the lipid phase is applied discontinuously as a layer on the applicator, at one or several sides of the applicator and this layer is dotted with particles of lipid phase material that are punched into the surface of the lipid layer by application of pressure. The material of the dots may be the same or different as that of the lipid layer.

The lipid phase preferably is applied in such manner that it will remain on the applicator surface during the manufacturing process and storage. This is conveniently accomplished by applying the lipid phase above its melting temperature, e.g. by spraying or coating it when molten to the surface of the applicator and subsequently allowing it to cool below its melting point so that the phase solidifies.

The lipid phase preferably is applied such that it is present at the surface of the applicator because of its physical location in that instance, the lipid phase is readily available to be spread onto the skin during usage. As a result, the effectiveness with which the lipid phase is transferred to the skin during use, the availability and therefore the effectiveness of any active ingredients incorporated therein is increased compared to products where the active agent is simply incorporated into a single continuously applied phase.

In preferred embodiments, the melting point or range of the lipid phase is above 25 °C, or within the temperature ranges specified above, because this allows to apply the lipid phase in liquid (molten) state to the applicator, and subsequently, after it having been cooled, to be present in solid state on the applicator. This allows a more convenient and easy after-treatment of the applicator to which the lipid phase has been applied in this manner, with the aqueous phase. In this way the two phases are be applied in such a manner that they do not mix or interact. In further preferred embodiments, the lipid phase is applied such that it forms a weak non-brittle film on the applicator. Applicators that have been treated this way are particularly stable, in particular during storing, essentially because mixing of the two phases is avoided in this way. Additionally such applicators will allow the lipid phase to melt upon contact with the skin, thus allowing a local mixing or emulsification of both phases.

In some embodiments of this invention the products may contain two or more lipid phases with different stability towards the aqueous phase. This allows one phase to interact more quickly with the aqueous phase than the other. This may find application in products where a gradual of active ingredient is desired or the release of a sequence of two or more active ingredients.

### The aqueous phase

The aqueous phase can be any of the art-known aqueous based formulations used to impregnate applicators. Beside water the aqueous phase may also contain further ingredients or additives such as surfactants, emulsifiers, consistency factors, conditioners, moisturizers, thickeners, preservatives, active ingredients, in particular cosmetic or dermatologically active ingredients, fragrances and the like. Active ingredients suited for topical applications are particularly preferred.

The aqueous phase may contain suitable dyes. In one type of embodiments, the lipid phase is applied discontinuously as a layer e.g. in the form of stripes leaving areas with only aqueous phase, which areas are colored. This allows the manufacture of applicator products with colored patterns, e.g. colored lines or even multicolored patterns when the lipid phase itself is also colored.

The aqueous phase may further contain lipophilic dyes, which upon contact with the lipid phase migrate into that phase and cause it to become colored.

The aqueous phase may further contain one or more preservatives such as, for example, phenoxyethanol, C₁-₄ alkylparabens and their salts, in particular their alkali metal salts such as sodium salts (e.g. C₁-₆ alkyl parabens such as methyl, ethyl, propyl, isopropyl, butyl paraben and the like parabens), chlorohexidine, formaldehyde or formaldehyde releaser, benzyl alcohol, chloroxylenol, phenoxyethanol, methylchloroisothiazolinone, methylisothiazolinone, sodium benzoate, chlorohexidine digluconate methyldibromo glutaronitrile, sodium borate, 5-bromo-5-nitro-1,3-dioxane, alcohol, benzoic acid, dehydroacetic acid, diazolidinyl urea, dichlorobenzyl alcohol, glucose oxidease, hexamidine diisethionate, imidazolidinyl urea, iodopropynyl butylcarbamate, isobutylparaben, isopropylparaben, lactoperoxidease, magnesium nitrate, PEG-4 laurate, phenethyl alcohol, polyaminopropyl biguanide, potassium sorbate, propylene glycol, pyridoxine HCl, quaternium-15, sorbic acid, triclosan, tocopherol and the like.

The aqueous phase may contain suitable surfactants, although preferably in limited amounts, e.g. less than 20%, or less than 15 %, or even less than 10 %, and in particular less than 5 % or less than 3 %, relative to the total weight of the aqueous phase. Examples of surfactants that can be incorporated are :
alkyl sulfates, e.g. sodium lauryl sulfate, ammonium lauryl sulfate, sodium cetearyl sulfate;
alkyl sulfoacetates, e.g. sodium lauryl sulfoacetate;
alkyl ether sulfates, e.g. sodium laureth sulfate, sodium trideceth sulfate, sodium oleth sulfate, ammonium laureth sulfate;
alkyl ether sulfosuccinates, e.g. disodium laureth sulfosuccinate;
alkyl glycosides e.g. decyl glucoside, lauryl glucoside;
alkyl isothionates;
amphoterics, e.g. cocamidopropyl betaine, sodium cocoamphoacetate, sodium lauroamphoacetate, disodium lauroamphodiacetate, disodium cocoamphodiacetate, sodium lauroamphopropionate, disodium lauroamphodipropionate, potassium or ammonium salts of the aforementioned amphoterics, capryl/capramidopropyl betaine, undecyleneamidopropyl betaine, lauramidopropyl betaine and fatty alcohol polyglycol ethers.

Suitable conditioners are e.g. alkylamido ammonium lactate, cetyl dimethicone, cetyl ricinoleate, dimethicone, laureth-23, laureth-4, polydecene, retinyl palmitate, agents selected from glyceryl monooleate and cocoglucoside including mixtures thereof (in particular the product 'Lamesoft ®' of Cognis which is a mixture of these two components), quaternized protein hydrolysates, quaternized cellulose and starch derivatives, quaternized copolymers of acrylic or methacrylic acid or salts, quaternized silicone derivatives, silicone oils, cyclomethicones, and the like agents, including mixtures thereof.

The aqueous phase may further contain suitable thickeners and film-forming substances.

The aqueous phase may contain pH sensitive components, i.e. components that change properties upon change of pH. The change of pH may occur when contacting the applicator product with the skin whereupon the pH changes from the pH of the product which usually is about pH 7 to the skin pH which is about pH 5.5. pH sensitive agents for example comprise particular emulsifiers, stabilizers, surfactants, viscosity regulating agents, chelators and the like.

In one type of embodiments an appropriate emulsifier is selected that is pH sensitive in this pH range in that it changes its emulsifying capacity, preferably increases its emulsifying capacity, so that upon contact with the skin an emulsification process occurs causing an interaction between the aqueous and lipid phases.

The above mentioned change of pH that occurs upon application of the product to the skin may also promote the release from active ingredients, in particular actives that are pH sensitive, e.g. actives having a pH dependent solubility.

### Application of the aqueous phase

The aqueous phase may be applied to the applicator using methods generally known in the art for applying aqueous liquid lotions such as spraying, dripping, immersing and the like techniques. A preferred application method for the aqueous phase is by spraying with a suitable nozzle or by drippling, for example by using a perforated tube with holes or slits. The immersing technique can be done by running the applicators through a bath holding the aqueous phase and subsequently controlling the amount of liquid that is absorbed by pressing.

The aqueous phase may be applied in various ways as described for the lipid phase, evenly or non-evenly, continuously or non continuously, at the surface or surface portion or, preferably, throughout the whole of the applicator material. Optionally some parts of the applicator can be left dry, i.e. not having the lipid and the aqueous phase, or some parts can only have the lipid or the aqueous phase. The lotion comprising the aqueous phase may be applied on the entire area of the applicator, either throughout the whole of the applicator or only at its surface portions. In the latter instance the aqueous phase may be applied at several sides or only at one side of the applicator.

The aqueous composition is typically applied in an amount of from about 1.0 g to 10 g per gram of substrate, preferably from 2.0 g to 5 g per gram of substrate, most preferably from 2 g to 4.5 g per gram of dry substrate, most preferably about 3.7 to about 3.8 g per gram substrate.

It may also be advantageous to only apply the lotion comprising the aqueous phase to only those areas (or that side) of the applicator which have (or has) not already been covered with the lipid phase.

Since in many cases the product is used as a cleansing article it is useful to design the aqueous phase as cleanser. Soils that are most difficult to clean are either water insoluble and/or strongly adhere to the skin. Therefore the aqueous phase is formulated such that it is capable of taking up water-insoluble materials.

### Further Phases

In another embodiment of the invention a third layer is applied to the applicator, which is made of polymeric material, hereafter referred to as polymeric layer. One or more polymeric layers may be applied to the applicator. Whenever used herein, the term 'polymeric layer' refers to one or more polymeric layers.

The polymeric layer may be applied to one side of the applicator or to several sides.

The polymeric layer is made of a suitable polymer such as polyethylene, polypropylene, polyester, a silicone and the like, including mixtures thereof. The polymeric layer may contain other materials, such as fillers or dyes. In the latter instance the area of the applicator covered with the polymeric layer will occur as colored areas. In case several polymeric layers are applied, layers with different colors may be used thus resulting in different colored patterns.

The polymeric layer may be applied to the applicator similarly as described for the application of the lipid phase. For example, it may be applied continuously, i.e. over the whole surface of the applicator, or discontinuously, e.g. in patterns, e.g. as stripes, spots or other figures. In the instance where the polymeric layer does not cover the whole surface, the lipid phase may cover both the areas of the applicator that are covered by the polymeric layer and the other areas.

The lipid layer may be applied onto the polymeric layer thus forming a double layer. The polymeric layer needs not be completely covered by the lipid phase, i.e. some parts may remain uncovered.

The polymeric layer may also be applied to the areas that are not covered by the lipid phase. For example the lipid phase may be applied as a layer in a discontinuous fashion and the polymeric phase is applied at the spots without lipid phase. In one particular embodiment the lipid phase is applied as stripes and the polymeric layer is put in the area between these stripes thus forming a pattern of alternating stripes of lipid phase and polymeric layer. This may for example be done at one side of the applicator while the aqueous phase is put at another side.

The polymeric layer may be semi-solid so that it can be disrupted upon application of a product having such a layer. Semi-solid polymeric layers are made of polymers that have a waxy, creamy or similar consistency. In that instance the polymeric layer can also be applied as an external coating onto the applicator, covering one or several sides, covering parts or the whole surface. It may also cover parts or the whole of the lipid layer.

The lipid phase that covers the polymeric layer may be colored or uncolored. In the former instance, the polymeric layer preferably is uncolored or white although it may be colored also. In the instance where the lipid phase is uncolored, the polymeric phase preferably is colored, although it may also be white or uncolored.

The polymeric phase may be applied for improving or promoting the transfer of the lipid phase that is coated thereon to the user's skin. Using a colored polymeric layer, or a colored lipid phase, or both, results in an appearance, disappearance or respectively change of color when the applicator product is used and the lipid phase is transferred to the skin.

The polymeric layer is applied to the applicator using art-known methods to coat materials used for manufacturing applicators with a polymeric layer. For example the polymeric layer can be applied by screen printing, gravure printing, roller printing, embossing, spraying, drippling, bathing and the like techniques.

### Additional ingredients for either one or both phases

The lipid and/or the aqueous phase may contain further ingredients that may be present in one or in both phases.

### Active ingredients

The lipid and/or the aqueous phase further may contain active ingredients for application to the skin. The lipid phase preferably contains oil-soluble or hydrophobic active agents, while the aqueous phase preferably contains water-soluble or hydrophilic active agents. However by using suitable emulsifiers oil-soluble or lipophilic active ingredients can be incorporated into the aqueous phase and vice versa, water-soluble or hydrophilic agents can be incorporated in the lipid phase.

Products having a lipid and/or an aqueous phase that contains one or more active ingredients constitute particularly attractive embodiments of the present invention. Preferred are those embodiments wherein the active ingredients are present in the lipid phase. The active ingredients can also be present in particular combinations.

The active ingredients, which may be lipophilic or hydrophilic, can be mixed with or incorporated into suitable carriers. These comprise any skin-acceptable inert materials that are known for formulating active ingredients. The carriers can be finely or more coarsely divided powders, or even granulates. They can comprise starches, sugars, binders, lubricants, diluents, fillers, disintegrating agents, granulating agents and the like components. The nature of the carrier materials will depend on the active ingredient that is formulated therein and on the type of formulation that is desired. Particular carriers for incorporating active ingredients are beads wherein the active ingredient is entrapped in some form. The terms 'beads' or 'polymeric beads' are meant to comprise any form of discrete, free-flowing powders, beads or capsules which envelope, coat or contain an active ingredient in a mono- or polymeric matrix or capsule. These terms are also meant to include porous beads or 'microsponges' and 'microcapsules', the latter being beads of smaller size. The beads may be coated with a suitable coating material that protects the interior of the bead or controls the release of the active ingredient entrapped therein. The coating on the bead itself may contain the active ingredient in which case the coating is layed on an inert core.

Formulating an active ingredient in beads can be for protecting the active from environmental factors but is mostly done for allowing controlled release of the active.

A particular type of beads are small beads or capsules, having an average diameter which is in the micrometer range, although the average diameter can be as small as even 200 nm.

This type of capsules can be liposome-based, made for example of phospholipids such as lecithin, phosphatidyl ethanolamine, phosphatidyl serine, phosphatidic acid and the like. This type of capsules also can be made of starch, cellulose, porous gelatin and the like.

The capsules or beads can also be relatively larger, having average sizes in the mm or 0.1 mm range. This type of capsules or beads can be made of materials such as agar, glycolic acid polymers, and further components such as water, mineral oils, glycerin. They may contain further ingredients such as preservatives, dye(s), and the like.

Another type of beads or microcapsules are microsponges. These are materials sized from about 5 to about 300 µm (average diameter) having a large inner surface. These are obtained by polymerization of particular monomers. An active ingredient can be entrapped therein either during this polymerization process or afterwards. Microsponge-based carriers may be used to protect the active ingredient entrapped therein or for controlled release purposes.

The capsules may optionally contain one or more suitable disintegrating agents, in particular those mentioned in this specification. Upon contact with the appropriate external factor, the disintegrating agents will cause the capsules to break open thus allowing release of the active ingredient entrapped therein.

The capsules can be incorporated into the lipid or the aqueous phase or into both. They can also be applied to the applicator prior to the introduction of the lipid and aqueous phase. They can even be introduced during the manufacturing process of the applicator itself.

Release of the active from the beads or capsules can be the result of the rupture of the coating or the matrix. This may be the result of physical factors such as pressure, strain or by shearing forces upon use of the applicator product, e.g. by rubbing the product to the skin or to a surface. Release of the active ingredient may be due to the semi-permeable or porous nature of the bead or its coating or due to external factors such as contact with liquid media that cause the active ingredient to become extracted, or to dissolve or disintegrate the bead or its coating, or by temperature effects. The capsules can also be disintegrated under influence of certain chemicals, in particular by disintegrating agents incorporated into the capsules. Particular embodiments of the latter are capsules containing suitable amounts of bicarbonate and an organic acid which, upon contact with water, e.g. upon contact with the aqueous phase when using the applicator product, cause the capsules to disintegrate.

The beads or capsules can be made according to methodologies generally known in the art, for example by emulsion polymerisation.

The beads or capsules may be applied to any portion of the applicator but preferably they are concentrated at the surface or in the upper surface portion of the applicator. This allows maximal transfer of the active ingredient to the skin or to the surface to which the product is applied.

The beads or capsules can be applied to the applicator in dry form by dusting, sifting, spraying and the like methods. They can also be printed or roll-coated in the form of a suitable liquid or paste. They can also be mixed with a suitable liquid, which can be a solvent that is inert towards the beads, or water, or the aqueous phase, and sprayed onto the applicator.

Examples of active agents for use in the aqueous or lipid phases comprise anti-microbials, e.g. anti-bacterials and antifungals, anti-inflammatory agents, anti-irritating compounds, anti-itching agents, moisturising agents, skin caring ingredients, plant extracts, vitamins, anti-inflammatories, actives for anti-stinging, anti-irritants, antidandruffs, anti-aging or anti-wrinkling agents, skin lifting agents such as dimethyl amino ethanol (DMAE) and in particular its salt-forms. Other suitable actives are e.g. Medicago officinalis, Actinidia chinensis, allantoin, Aloe barbadensis, Anona cherimolia, Anthemis nobilis, Arachis hypogaea, Arnica montana, Avena sativa, beta-carotene, bisabolol, Borago officinalis, butylene glycol, Calendula officinalis, Camellia sinensis, camphor, Candida bombicola, capryloyl glycine, Carica papaya, Centaurea cyanus, cetylpyridinium chloride, Chamomilla recutita, Chenopodium quinoa, Chinchona succirubra, Chondrus crispus, Citrus aurantium dulcis, Citrus grandis, Citrus limonum, Cocos nucifera, Coffea arabica, copper peptides such as copper tripeptide-1, Crataegus monogina, Cucumis melo, dichlorophenyl imidazoldioxolan, Enteromorpha compressa, Equisetum arvense, ethoxydiglycol, ethyl panthenol, farnesol, ferulic acid, Fragaria chiloensis, Gentiana lutea, Ginkgo biloba, glyceryl laurate, Glycine soya, Glycyrrhiza glabra, Hamamelis virginiana, heliotropine, hydrogenated palm glycerides, citrates, hydrolyzed castor oil, hydrolyzed wheat protein, Hypericum perforatum, Iris florentina, Juniperus communis, lactis proteinum, lactose, Lawsonia inermis, linalool, Linum usitatissimum, lysine, Magnesium aspartate, magnifera indica, Malva sylvestris, mannitol, mel, Melaleuca alternifolia, Mentha piperita, menthol, menthyl lactate, Mimosa tenuiflora, Nymphaea alba, olaflur, Oryza sativa, panthenol, paraffinum liquidum, PEG-20M, PEG-26 jojoba acid, PEG-26 jojoba alcohol, PEG-35 castor oil, PEG-40 hydrogenated castor oil, PEG-60 hydrogenated castor oil, PEG-8 caprylic/capric acid, Persea gratissima, petrolatum, potassium aspartate, potassium sorbate, propylene glycol, Prunus amygdalus dulcis, prunus armeniaca, Prunus persica, retinyl palmitate, Ricinus communis, Rosa canina, Rosmarinus officinalis, Rubus idaeus, salicylic acid, Sambucus nigra, sarcosine, Serenoa serrulata, Simmondsia chinensis, sodium carboxymethyl betaglucan, sodium cocoyl amino acids, sodium hyaluronate, sodium palmitoyl proline, stearoxytrimethylsilane, stearyl alcohol, sulfurized TEA-ricinoleate, talcum, thymus vulgaris, Tilia cordata, tocopherol, tocopheryl acetate, trideceth-9, Triticum vulgare, tyrosine, undecylenoyl glycine, urea, Vaccinium myrtillus, valine, zinc oxide, zinc sulfate and the like.

Of particular interest are active ingredients, that can be used for treating skin that shows inflammatory reactions, that is irritated, red or damaged. Examples of such agents are zinc compounds or sulphur.

Further active ingredients that can be used are known under the tradename Generol^{™}. These comprise ethoxylated and non-ethoxylated phytosterines. Other active ingredients comprise anti-microbial agents and biogenic active ingredients.

The active ingredients can be present, depending on the nature of the ingredients and their application, in various concentrations, but usually are present in a quantity in the range of 0.01-10 % (w/w), preferably from 0.1- 7 % (w/w) and more preferably 1-5 % (w/w) w/w expressed to the total weight of the lipid and the aqueous phase.

### Further additional ingredients

Both phases may contain further ingredients such as moisturizers, refattening agents, thickeners, powders, biogenic actives, deodorants, film formers, UV sunscreen filters, anti-oxidants, hydrotropes, preservatives, insect repellents, self tanners, solubilizers, perfumes, dyes, pigments, and the like.

### Moisturizers

The lipid and/or aqueous phase can further contain one or more moisturizers. These are added to improve the sensoric properties as well as to regulate skin hydratation. These agents additionally can improve the penetration of the composition in or into the applicator, in particular in or into the applicators.

Moisturizers typically may be present in quantities of 1-20 % (w/w), preferably of 5 - 15 % (w/w), and more preferably 5 -10 % (w/w) - relative to the total amount of the lipid and/or the aqueous phase.

Suitable moisturizers are a.o. amino acids, pyrrolidone carbonic acid, lactic acid and its salts, lactitol, urea and urea derivatives, ureic acid, glucosamine, creatinine, hydrolysis products of collagen, chitosan or chitosan salts/-derivatives, and in particular polyols and polyol derivatives (e.g. ethylene glycol, propylene glycol, butylene glycol, pentylene glycol, hexylene glycol, erythrite, 1,2,6-hexanetriol, polyethylene glycols such as PEG-4, PEG-6, PEG-7, PEG-8, PEG-9, PEG-10, PEG-12, PEG-14, PEG-16, PEG-18, PEG-20, PEG-135, PEG 150), sugar and sugar derivatives (a.o. fructose, glucose, maltose, maltitol, mannite, inosite, sorbite, sorbityl silandiol, sucrose, trehalose, xylose, xylit, glucuronic acid and its salts), ethoxylated sorbitol (Sorbeth-6, Sorbeth-20, Sorbeth-30, Sorbeth-40), honey and hydrogenated honey, hydrogenated starch hydrolysates, as well as mixtures of hydrogenated wheat protein, hydrolyzed milk protein, lecithin, pythantriol, hyaluronic acid and salts thereof, and PEG-20-acetate copolymers. Particularly preferred moisturizers are glycerine, diglycerine and triglycerine.

The products according to this invention can be used as anti-perspirants or deodorants. In products for these applications either one or both phases contain actives that have deodorizing and /or anti-perspirant properties.

The products according to the invention can also be used in sunscreen applications and in that instance take the form of sunscreen applicators. In these products the lipid and/or aqueous phase contains one or more sunscreen filters which are for example organic substances that are capable of absorbing ultraviolet radiation and to set free the absorbed energy as longer-wave radiation, e.g. as thermic energy. UVB-filters can be oil or water-soluble. Insoluble suncreen pigments, namely finely dispersed metal oxides or metal salts can further be added, as well as secondary light protecting factors. Further agents that may be added include hydrotropes, self-tanning agents, perfume oils, dyes.

The addition of a dye has the advantage that it provides of a visible indication for the user, sending the message of particular (active) ingredients having been incorporated in the lipid phase. It allows furthermore to visualize the stability of the phase, in particular of the lipid phase, that has been applied on the applicator can be easily visualized. This allows, for example, to monitor whether the oily and aqueous phases have become mixed upon the storage.

### Emulsifiers

The lipid and/or aqueous phase in the products of the invention may further contain one or more emulsifiers which can be of the W/O (for use in the lipid phase) or the O/W (for use in the aqueous phase) type. The addition of an emulsifier allows the incorporation of hydrophilic components or agents into the lipid phase and vice versa of lipophilic components or agents into the aqueous phase.

Preferred are non-ionic emulsifiers which typically have good skin compatibility. Improved sensoric properties are obtained when combining non-iononics W/O and O/W emulsifiers. The lipid and/or aqueous phase may contain the emulsifier(s) in an amount of 0 to 20 % (w/w), in particular of 0.1 to 15 % (w/w), more in particular of 0.1 to 10 % (w/w), still more in particular from 0.1 to 5%, or 0.1 to 2% (w/w), relative to the total quantity of the lipid and/or aqueous phase.

### Non-ionic emulsifiers

Particular non-ionic emulsifiers comprise:
(1) Addition products of 2 to 50 moles of ethylene oxide and/or 0 to 20 moles propylene oxide to linear fatty alcohols having 8 to 40 C-atoms, to fatty acids with 12 to 40 C-atoms and to alkylphenols with 8 to 15 C-atoms in the alkyl rest.
(2) C_{12/18}-fatty acid mono- and -diesters of addition products of 1 to 50 moles of ethylene oxide and glycerine.
(3) Glycerine mono- and -diesters and sorbitan mono- and -diesters of saturated and unsaturated fatty acids with 6 to 22 C-atoms and their ethylene oxide addition products.
(4) Alkyl mono- and -oligoglycosides with 8 to 22 C-atoms in the alkyl rest and their ethoxylated analogs.
(5) Addition products of 7 to 60 moles of ethylene oxide to castor oil and/or hardened castor oil.
(6) Polyol- and in particular polyglycerine esters, such as e.g. polyol poly-12-hydroxystearate, polyglycerine polyricinoleate, polyglycerine diisostearate or polyglycerine dimerate. Also applicable are mixtures of compounds of several of these substance classes.
(7) Addition products of 2 to 15 moles of ethylene oxide to castor oil and/or hardened castor oil.
(8) Partial esters derived from linear, branch chained, unsaturated or saturated C₆-C₂₂-fatty acids, ricinoleic acid as well as 12-hydroxystearic acid and glycerine, polyglycerine, pentaerythrite, dipentaerythrit, sugar alcohols (e.g. sorbitol), alkylglucosides (e.g. methylglucoside, butylglucoside, laurylglucoside) as well as polyglucosides (e.g. cellulose), or mixed esters such as e.g. glyceryl stearate/citrate and glyceryl stearate/lactate.
(9) Wool wax alcohols.
(10) Polysiloxane-polyalkyl-polyether-copolymers and derivatives thereof.
(11) Mixed esters from pentaerythrite, fatty acids, citric acid and fatty alcohols and/or mixed esters of fatty acids with 6 to 22 C-atoms with methylglucose and polyoles, respectively glycerine or polyglycerine.
(12) Polyalkylene glycols.

The addition products of ethylene oxide and/or of propylene oxide and fatty alcohols, fatty acids, alkylphenoles, glycerine mono- and -diesters as well as sorbitan mono- and -diesters of fatty acids or of castor oil are known and commercially available products. Usually these are mixtures of homologues of which the average degree of alkoxylation corresponds to the ratio of starting quantities of ethylene oxide and/or propylene oxide and substrate, with which the addition reaction is conducted. Depending upon the degree of alkoxylation these products are either W/O- or O/W-emulsifiers. C_{12/18}-fatty acid mono- and -diesters of addition products of ethylene oxide to glycerine are known as re-fatting agents in cosmetic applications.

Particular useful and mild emulsifiers are polyolpoly-12-hydroxystearates and mixtures thereof with other components, that are available under the tradename "Dehymuls^{®}" PGPH" (W/O-emulsifier) or "Eumulgin^{®} VL 75" (1:1 w/w mixture with coco-glucosides, O/W-emulsifier) or Dehymuls^{®} SBL (W/O-Emulsifier) from Cognis Deutschland GmbH. The polyol components of these emulsifiers can be derived from materials that have at least two and in particular 3 to 12 and more in particular 3 to 8 hydroxyl groups, and 2 to 12 carbon atoms.

In case it is desirable to incorporate water-soluble active ingredients and/or small amounts of water into the lipid phase it can be advantageous to add an emulsifier selected from the group of non-ionic O/W-emulsifiers (HLB-value: 8-18) and/or solubilizers. These can for example be the already mentioned ethylene oxide-adducts with a corresponding high degree of ethoxylation e.g. 10 - 20 ethylene oxide units in the case of O/W-emulsifiers and 20 - 40 ethylene oxide units for so-called solubilizers. Particularly attractive as O/W emulsifiers are Ceteareth-12 und PEG-20 stearate. Particularly attractive solubilizers are Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether) and Eumulgin^{®} SML 20 (INCI: Polysorbate-20).

Non-ionic emulsifiers of the group of alkyl oligoglycoside are particularly skin-compatible and therefore preferred as O/W-Emulsifiers. C₈-C₂₂-alkyl mono- and - oligoglycosides, their preparation and use have been described in the prior art. Oligoglycosides are meant to comprise oligomeric glycosides with a degree of oligomerisation of up to about 8. The degree of oligerisation can also be a statistical average used for those products comprised of a specific range of oligoglycosides. An example is the product sold under the tradename Plantacare^{®} which has a C₈-C₁₆-alkyl group glycosidically bound to an oligoglucoside rest, having an average degree of oligomerisation between 1 and 2.

Other non-ionic emulsifiers are the acyl glucamides. Preferred is the product sold under the tradename Emulgade^{®} PL 68/50 (Cognis Deutschland GmbH) which is a 1:1-mixture of alkyl polyglucosides and fatty alcohols, and a mixture of lauryl glucoside, polyglyceryl-2-dipolyhydroxystearate, glycerine and water, sold under the trade name Eumulgin^{®} VL 75.

Lipophilic W/O-emulsifiers in principle are emulsifiers with a HLB-value in the range of 1 to 8. The HLB-value of ethoxylated products is calculated by the formula: HLB = (100 - L) : 5, wherein L is the percentage (in weight %) of lipophilic groups, i.e. of fatty alkyl- or fatty acyl groups in the ethylene oxide adducts.

Particularly attractive W/O-emulsifiers are the partial esters of polyoles, in particular of mono-, di- or tri-, sesqui esters of fatty acids of polyoles, more in particular of C₃-C₆-polyoles, such as, for example, glyceryl monoesters, partial esters of pentaerythrite or carbohydrate esters, e.g. saccharose distearate, or sorbitan mono-, di-, tri- or sesqui fatty esters in particular stearates, oleates, erucates, ricinoleates, hydroxystearates, isostearates (but also: tartrates, citrates, maleates) and the like. Also attractive are addition products of 1 to 30, respectively 5 to 10 moles ethylene oxide to these sorbitan esters.

### Further Surfactants/Emulsifiers for both phases

Depending upon the use of the products of the present invention, the lipid and/or aqueous phase may further contain zwitterionic, amphoteric, cationic and or anionic surfactants.

Zwitterionic surfactants are those tensioactive compounds, that contain at least a quaternary ammonium group and at least a -COO⁽⁻⁾- or -SO₃⁽⁻⁾- group. Particularly useful zwitterionic surfactants are the so-called betaines such as N-alkyl-N,N-dimethyl ammonium glycinate, for example coco-alkyl dimethylammonium glycinate, N-acyl-aminopropyl-N,N-dimethylammonium glycinate, for example coco-acyl aminopropyl dimethylammonium glycinate, and 2-alkyl-3-carboxylmethyl-3-hydroxyethylimidazoline, each having 8 to 18 C-atoms in the alkyl- or acyl group as well as coco-acyl aminoethyl hydroxyethyl carboxymethyl glycinate. A preferred zwitterionic surfactant is the fatty acid amide-derivative known by its INCI-name cocamidopropyl betaine.

Ampholytic surfactants can further be added, in particular as co-surfactants. Ampholytic surfactants comprise those tensioactive compounds, that beside a C₈-C₁₈-alkyl- or acyl group at least contain a free amino group and at least a -COOH- or -SO₃H- group and are able to form internal salts. Examples of appropriate ampholytic surfactants are N-alkyl glycines, N-alkyl propionic acids, N-alkyl amino buteric acids, N-alkyl iminodipropionic acids, N-hydroxyethyl-N-alkyl amidopropyl glycines, N-alkyl taurine, N-alkyl sarcosine, 2-alkylaminopropionic acids and alkylamino acetic acids with in each alkyl group about 8 to 18 C-atoms.

Most preferred ampholytic surfactants N-coco-alkyl aminopropionate coco-acyl amino ethylamino propionate and C₁₂₋₁₈-acylsarcosine.

Anionic surfactants are characterized by a water solubilizing anionic group such as a carboxylate-, sulfate-, sulfonate- or phosphate- group and a lipophilic rest. Particular anionic surfactants are the alkali-, ammonium- or alkanol ammonium salts of alkyl sulfates, alkyl ethersulfates, alkyl ethercarboxylates, acyl isethionates, acyl sarkosinates, acyl taurines with linear alkyl- or acyl groups having 12 to 18 C-atoms as well as alkali- or ammonium salts of sulfosuccinates and acyl glutamates.

Quaternary ammonium derivatives can in particular be used as cationic surfactants. Preferred are ammonium halogenides, in particular chlorides and bromides, e.g. alkyl trimethylammonium chloride, dialkyl dimethylammonium chloride and trialkyl methylammonium chloride, z. B. cetyl trimethylammonium chloride, stearyl trimethylammonium chloride, distearyl dimethylammonium chloride, lauryl dimethylammonium chloride, lauryl dimethylbenzylammonium chloride and tricetyl methylammonium chloride. Additional cationic surfactants are the quaternary esters with good biological degradability, such as, for example, dialkylammonium methosulfates and methylhydroxyalkyl dialkoyloxy alkylammonium methosulfates (sold under the tradename Stepantex^{®} and the products of the Dehyquart^{®}-series). The term "Esterquats" is meant to comprise quaternized fatty acid triethanolamine ester salts which have a beneficial impact on the softness of the phases, in particular of the lipid phase. Further cationic surfactants are the quaternized protein hydrolysates.

### Manufacture.

This invention further concerns a process for preparing a product as defined herein, in accordance with the features of claim 19. Said process comprises contacting the applicator with a lipid phase composition and subsequently an aqueous phase composition as described herein and drying the product. The process comprises contacting the applicator with the lipid phase and subsequently with the aqueous phase and drying the product.

Contacting the applicator with the aqueous phase comprises impregnating it with the aqueous phase by procedures such as, for example, running through a bath, immersing, spraying, drippling and the like techniques. Contacting the applicator with the lipid phase is as described above in the section 'lipid phase', preferably by spraying, printing or by a direct contact procedure in which there is a direct contact between the applicator and an application head having slit nozzles.

The drying step may be applied at any time during the process, but it should be after the application of the aqueous phase. Drying can be done by conventional methods, e.g. by the application of hot air, or by leading the wet applicator through an oven or over an heated or warmed transport roll. In case a lipid phase has been applied prior to drying, the temperature of the air should be such that the lipid phase does not melt. Application of air of ambiant temperature may be recommendable in that instance.

The process comprises contacting the applicator with a lipid phase and subsequently with an aqueous phase, whereafter the thus obtained product is dried.

The lipid and aqueous phases can also be applied to the applicator at any time during the manufacturing process of the applicator, for example either one or both of the phases may be applied during the manufacturing process of the applicator material. Preferably the lipid and/or aqueous phase are applied to the applicator after finishing the manufacturing process of the applicator, more preferably after the applicator has been dried.

The thus obtained applicators can be packed individually or can be packed in a determined number, e.g. a number between 10 and 30 in a suitable package, for example a plastic wrap, box and the like.

Applicators with different coating and/or impregnation can be combined in one packaging. For example there can be a series of applicators with increasing or decreasing amounts of lipid phase. Or colored or uncolored applicators can be alternated.

### Application and properties

The products according to the present invention advantageously result in an optimal release of the active ingredient(s), in particular when incorporated in the lipid phase, onto the skin during use.

Optimal release of active ingredients can be achieved by using a lipid phase which is a solid lipid having a melting point or melting range which is equal to or slightly exceeds body temperature. Without being bound to theory, it is believed that this results in a quicker melting of the lipid phase causing a faster and more efficient transfer and release to the skin of the active materials.

Optimal release of active ingredients can also be achieved by using a suitable emulsifier in one or both of the phases to cause a local emulsification process on the skin during use of the applicators. Preferably the emulsifier is present in the aqueous phase. This local emulsification may be the result of body temperature causing the lipid phase to melt or it may be the result of pressure exerted during usage of the applicator, or it may be the result of both, the latter being usually the case. In the instance of local emulsification by the effect of pressure, the emulsification process is driven by the (limited) pressure exerted by the user when applying the applicator, e.g. by rubbing it across the skin, dabbing it and the like. This causes the lipid phase to come in contact with water or an aqueous phase and form an emulsion locally. This local emulsification can also be achieved by contacting the lipid phase in the products with water or with an aqueous phase prior to usage. Or this local emulsification is achieved by using the products on a wet skin.

In this local emulsification process, a limited amount of the phase without emulsifier is incorporated into the phase having the emulsifier. In preferred embodiments, the aqueous phase contains a small amount of emulsifier, for example the emulsifier may be present in an amount from about 0.5 to about 5%, more in particular from about 1 to about 3%. In that instance some of the lipid phase is locally emulsified into the aqueous phase.

Although in preferred embodiments the lipid phase is not present on the whole surface of the applicator, nevertheless a good release of the lipid phase and of the components contained therein is attained, especially when the local emulsification process comes into play.

Optimal release of active ingredients can also be achieved by making use of both above possibilities.

The products of the invention mainly are aimed for use as end products. In this instance the consumer is instructed to treat these products with water or with an aqueous lotion which for example be sold separately.

Or they can be used as such, e.g. as a dry applicator for use on a wet skin.

The products according to the invention can be for baby or adult use in a wide range of applications as personal care products, comprising, for example, baby cleansing applicator, face or body cleansing applicator, applicator skin treatment or skin conditioning such as for example skin moisturization and against skin aging, insect repellence applicator, powder applicators, toilet applicators, anti-perspirant applicators, peeling applicators, after-sun treatment, sunscreen applicators, applicators for feminine hygiene, nappy rash applicators, the latter preferably containing zinc oxide as active ingredient, and the like.

The products of the present invention have a low water content, for example a water content which is below 10 %, or lower w/w relative to the total weight of the product. Examples of products with low water content are the so-called dry applicators which are aimed for use on a wet skin. Examples of applications for this type of applicators are usage in the shower or after bathing. Such dry applicators may also be recommended for use after wetting the product itself, e.g. with water or with an aqueous lotion that is provided separately.

Examples of products with relatively higher water content from about 5 % to about 10 % are so-called intermediate dry applicators.

The products of the invention may find use as cleansing tools, in particular when wetted, however their use is not limited to this application only. They are particularly effective when wetted with water, which is due, i.a., by the fact that they can remove both aqueous and lipid soils and components. The products of the invention may in particular be used as cleansers for babies because of their effectiveness to remove waste deposits as well as to reduce the number of microorganisms that can cause infection.

The products described herein find use as applicators of active substances, in particular of the active substances mentioned herein, or they find use as both cleanser and applicator of active substances in one product.
The products of this invention have excellent transfer of active ingredients to the skin thus widening the applications of applicator products as a vehicle for a number of actives, in particular more expensive actives that so far could not be applied because of poor transfer rate. The products of this invention not only provide a more efficient transfer of active ingredients to the skin, but moreover provide other consumer benefits such as a more even distribution of the actives on the skin, better skin penetration.

The products of this invention show the additional advantage that they may combine in one and the same product both cleansing capability and the transfer of active ingredients to the skin, i.e. the application of leave-on products. They further allow to independently optimize the cleansing and skincare attributes of the product and at the same time improve the delivery of skincare actives onto the skin. Hence,either of both aspects may be present in a larger extend, i.e. the product may be primarily for cleansing purposes but also having the capability of transferring certain beneficial components or active substances to the skin, or vice versa, the products may be designed for applications in instances where the primary benefit is not cleansing but a better and more convenient form of application of leave-on products.

The products of the invention may furthermore have improved performance in terms of cleansing and skin benefits since both attributes can be formulated in different phases independently.

Another benefit of the products of this invention is that they may offer a softer feel of the applicator material due to the modification of the applicator surface caused by the presence of the lipid phase. The products moreover offer gentler cleansing because of less friction of the applicator on the skin (softer skin-feel).

A still further advantage lies in the fact that the instant products allow an improved transfer of actives onto the skin since the active ingredients usually are concentrated at the surface of the applicator material and not included in the inner phase of a typical o/w-emulsion.

Most types of lipid and aqueous phases described herein possess the additional advantage that they are almost odorless (unless fragrances are added), environmentally friendly and biologically decomposable.

The products of this invention are particularly attractive because they allow convenient and quick application and allow an easy and more evenly distribution of any ingredient incorporated therein or thereon. They moreover are convenient for application on babies and children. The products additionally allow faster and effective cleansing.

In view of these beneficial properties, the products of this invention can be used in a wide variety of cosmetic and personal care applications, but also in other cleaning or cleansing applications such as cleaning of hard surfaces.

### Examples

The following examples are given with the nomenclature of INCI. As used in the following examples, C.I. refers to dyes.

### Example 1: lipid phases

| Phase 1-A | |
|---|---|
| Cocoglycerides | 64.99 % |
| Cetyl Alcohol | 33.00 % |
| Di-Stearyl Ether | 1.00 % |
| Tocopherol | 1.00 % |
| C.I. 61565 | 0.01 % |
| | |

| Phase 1-B | |
|---|---|
| Cocoglycerides | 54.99 % |
| Cetyl Alcohol | 33.00 % |
| Ceteareth-12 | 3.00 % |
| Glyceryl Stearate | 4.00 % |
| Di-Stearyl Carbonate | 2.00 % |
| Tocopherol | 1.00 % |
| C.I. 61565 | 0.01 % |
| Aqua | 2.00 % |
| | |

| Phase 1-C | |
|---|---|
| Cocoglycerides | 49.99 % |
| Cetearyl Alcohol | 20.00 % |
| Cegesoft@ HF 52 | 5.00 % |
| Cegesoft® PS 6 | 3.00 % |
| Ceteareth-12 | 2.00 % |
| Glyceryl Stearate | 2.00 % |
| PEG-20 Stearate | 10.00 % |
| Di-Stearyl Ether | 2.00 % |
| Tocopherol | 1.00 % |
| C.I. 61565 | 0.01 % |
| Aqua | 5.00 % |

| Phase 1-D | |
|---|---|
| Cocoglycerides | 58.99 % |
| Glyceryl Stearate | 25.00 % |
| Glyceryl Laurate | 14.00 % |
| Di-Stearyl Carbonate | 1.00 % |
| Tocopherol | 1.00 % |
| C.I. 75300 | 0.01 % |
| | |

| Phase 1-E | |
|---|---|
| Cocoglycerides | 30.00 % |
| Cetearyl Alcohol | 1.00 % |
| Cegesoft® HF 52 | 20.00 % |
| Cegesoft® GPO | 5.00 % |
| Ceteareth-12 | 15.00 % |
| Glyceryl Stearate | 20.00 % |
| Di-Stearyl Ether | 5.00 % |
| Tocopherol | 1.00 % |
| Panthenol | 1.00 % |
| Aqua | 2.00 % |
| | |

| Phase 1-F | |
|---|---|
| Cocoglycerides | 19.99 % |
| Cetearyl Alcohol | 30.00 % |
| Cegesoft® PS 6 | 10.00 % |
| Eumulgin® VL 75 | 10.00 % |
| Ceteareth-12 | 5.00 % |
| Glyceryl Stearate | 10.00 % |
| Di-Stearyl Carbonate | 5.00 % |
| Tospearl® 145 A | 5.00 % |
| Zinc Stearate | 2.00 % |
| C.I. 61565 | 0.01 % |
| Aqua | 3.00 % |

| Phase 1-G | |
|---|---|
| Myristyl Alcohol | 19.99 % |
| Cocoglycerides | 10.00 % |
| Cegesoft® HF 52 | 20.00 % |
| Eumulgin® VL 75 | 10.00 % |
| Glyceryl Stearate | 20.00 % |
| PEG-20 Stearate | 5.00 % |
| Di-Stearyl Carbonate | 2.00 % |
| Panthenol | 3.00 % |
| C.I. 61565 | 0.01 % |
| Aqua | 10.00 % |
| | |

| Phase 1-H | |
|---|---|
| Myristyl Alcohol | 47.99 % |
| Stearyl Alcohol | 25.00 % |
| Eumulgin® VL 75 | 2.00 % |
| PEG-20 Stearate | 14.00 % |
| 1,2-Hexadecanediol | 5.00 % |
| Bisabolol | 1.00 % |
| C.I. 47000 | 0.01 % |
| Aqua | 5.00 % |
| | |

| Phase 1-I | |
|---|---|
| Cocoglycerides | 47.99 % |
| Stearyl Alcohol | 20.00 % |
| Eumulgin® VL 75 | 2.00 % |
| PEG-20 Stearate | 12.00 % |
| Di-Stearyl Carbonate | 5.00 % |
| Cyclomethicone | 3.00 % |
| Tospearl® 145 A | 5.00 % |
| C.I. 75300 | 0.01 % |
| Aqua | 5.00 % |

| Phase 1-J | |
|---|---|
| Cocoglycerides | 55.99 % |
| Glyceryl Stearate | 20.00 % |
| Glyceryl Laurate | 15.00 % |
| Di-Stearyl Carbonate | 5.00 % |
| Talc | 2.00 % |
| Aluminum Starch Octenylsuccinate | 2.00 % |
| C.I. 60725 | 0.01 % |
| | |

| Phase 1-K | |
|---|---|
| Cocoglycerides | 50.99 % |
| Glyceryl Stearate | 25.00 % |
| Glyceryl Laurate | 15.00 % |
| Di-Stearyl Ether | 5.00 % |
| Talc | 2.00 % |
| Timiron® Splendid Gold | 2.00 % |
| C.I. 21230 | 0.01 % |
| | |

| Phase 1-L | |
|---|---|
| Myristyl Alcohol | 58.99 % |
| Stearyl Alcohol | 23.00 % |
| PEG-20 Stearate | 15.00 % |
| Di-Stearyl Carbonate | 2.00 % |
| Panthenol | 1.00 % |
| C.I. 61525 | 0.01 % |
| | |

| Phase 1-M | |
|---|---|
| Myristyl Alcohol | 47.99 % |
| Stearyl Alcohol | 25.00 % |
| Eumulgin® VL 75 | 2.00 % |
| PEG-20 Stearate | 10.00 % |
| Di-Stearyl Ether | 7.00 % |
| Panthenol | 2.00 % |
| C.I. 61525 | 0.01 % |
| Aqua | 6.00 % |
| | |
| Phase 1-N | |
| Myristyl Alcohol | 50.00 % |
| Stearyl Alcohol | 25.00 % |
| Eumulgin® VL 75 | 2.00 % |
| PEG-20 Stearate | 10.00 % |
| Di-Stearyl Ether | 7.00 % |
| Ethyl Butylacetylaminopropionate | 5.00 % |
| Panthenol | 1.00 % |
| | |
| Phase 1-O | |
| Cocoglycerides | 54.99 % |
| Cetyl Alcohol | 33.00 % |
| Ceteareth-12 | 3.00 % |
| Glyceryl Stearate | 4.00 % |
| Di-Stearyl Carbonate | 2.00 % |
| Octyl Methoxycinnamate | 6.00 % |
| C.I. 61565 | 0.01 % |
| | |
| Phase 1-P | |
| Cocoglycerides | 56.99 % |
| Glyceryl Stearate | 25.00 % |
| Glyceryl Laurate | 14.00 % |
| Di-Stearyl Carbonate | 1.00 % |
| Polyethylene | 3.00 % |
| C.I. 75300 | 0.01 % |

| Phase 1-Q | |
|---|---|
| Cocoglycerides | 58.93 % |
| Glyceryl Stearate | 25.00 % |
| Glyceryl Laurate | 15.00 % |
| Di-Stearyl Ether | 1.00 % |
| Aqua | 0.06 % |
| C.I. 61565 | 0.01 % |
| | |

| Phase 1-R | |
|---|---|
| Cocoglycerides | 43.93 % |
| Stearyl Alcohol | 15.00 % |
| Glyceryl Stearate | 25.00 % |
| Glyceryl Laurate | 15.00 % |
| Di-Stearyl Ether | 1.00 % |
| Aqua | 0.06 % |
| C.I. 61565 | 0.01 % |
| | |

| Phase 1-S | |
|---|---|
| Cocoglycerides | 44.93 % |
| Glyceryl Stearate | 25.00 % |
| Glyceryl Laurate | 15.00 % |
| Di-Stearyl Ether | 15.00 % |
| Aqua | 0.06 % |
| C.I. 61565 | 0.01 % |

### Example 2: aqueous phases

| Phase 2-A | |
|---|---|
| Aqua | 96.336 % |
| Polysorbate 20 | 0.600 % |
| PEG-75 Lanolin | 0.100 % |
| Parfum | 0.150 % |
| PEG-40 Hydrogenated Castor Oil | 0.400 % |
| Propylene Glycol | 1.120 % |
| Phenoxyethanol | 0.800 % |
| Tetrasodium EDTA | 0.078 % |
| Chamomilla Recutita | 0.070 % |
| Ethoxydiglycol | 0.171 % |
| Butylene Glycol | 0.035 % |
| Glucose | 0.016 % |
| Iodopropynyl Butylcarbamate | 0.010 % |
| PEG- 4 Laurate | 0.090 % |
| Citric Acid | 0.020 % |
| | |

| Phase 2-B | |
|---|---|
| Aqua | 98.252 % |
| Phenoxyethanol | 0.800 % |
| Iodopropynyl Butylcarbamate | 0.010 % |
| PEG- 4 Laurate | 0.090 % |
| Parfum | 0.150 % |
| Tetrasodium EDTA | 0.078 % |
| Citric Acid | 0.020 % |
| Polysorbate 20 | 0.600 % |

| Phase 2-C | |
|---|---|
| Aqua | 97.250 % |
| Glycerines | 1.000 % |
| Phenoxyethanol | 0.800 % |
| Iodopropynyl Butylcarbamate | 0.010 % |
| PEG- 4 Laurate | 0.090 % |
| Parfum | 0.150 % |
| Tetrasodium EDTA | 0.078 % |
| Citric Acid | 0.020 % |
| Polysorbate 20 | 0.600 % |
| | |

| Phase 2-D | |
|---|---|
| Aqua | 96.332 % |
| Glycerines | 1.000 % |
| Phenoxyethanol | 0.800 % |
| Polysorbate 20 | 0.600 % |
| PPG-15 Stearyl Ether | 0.400 % |
| PEG-7 Glyceryl Cocoate | 0.100 % |
| Propylene Glycol | 0.350 % |
| Iodopropynyl Butylcarbamate | 0.010 % |
| PEG- 4 Laurate | 0.090 % |
| Chamomilla Recutita | 0.070 % |
| Parfum | 0.150 % |
| Tetrasodium EDTA | 0.078 % |
| Citric Acid | 0.020 % |
| | |

| Phase 2-E | |
|---|---|
| Aqua | 97.33 % |
| Phenoxyethanol | 0.800 % |
| Polysorbate 20 | 0.600 % |
| Sorbeth-30 | 0.400 % |
| Propylene Glycol | 0.350 % |
| Dimethicone Copolyol | 0.100 % |
| Iodopropynyl Butylcarbamate | 0.010 % |
| PEG- 4 Laurate | 0.090 % |
| Chamomilla Recutita | 0.070 % |
| Parfum | 0.150 % |
| Tetrasodium EDTA | 0.078 % |
| Citric Acid | 0.020 % |
| | |

| Phase 2-F | |
|---|---|
| Aqua | 97.332 % |
| Phenoxyethanol | 0.800 % |
| PEG-80 Sorbitan Laurate | 0.600 % |
| Propylene Glycol | 0.350 % |
| Sorbeth-30 | 0.400 % |
| Octyldecanol | 0.100 % |
| Iodopropynyl Butylcarbamate | 0.010 % |
| PEG-4 Laurate | 0.090 % |
| Chamomilla Recutita | 0.070 % |
| Parfum | 0.150 % |
| Tetrasodium EDTA | 0.078 % |
| Citric Acid | 0.020 % |
| | |

| Phase 2-G | |
|---|---|
| Aqua | 97.332 % |
| Phenoxyethanol | 0.800 % |
| Polysorbate-20 | 0.600 % |
| PGG-15 Stearyl Ether | 0.400 % |
| Propylene Glycol | 0.350 % |
| Decyl Oleate | 0.100 % |
| Iodopropynyl Butylcarbamate | 0.010 % |
| PEG-4 Laurate | 0.090 % |
| Chamomilla Recutita | 0.070 % |
| Parfum | 0.150 % |
| Tetrasodium EDTA | 0.078 % |
| Citric Acid | 0.020 % |
| | |

| Phase 2-H | |
|---|---|
| Sodium Myreth Sulfate | 10.00 % |
| Lauryl Glucoside | 15.00 % |
| Cocamidopropyl Betaine | 10.00 % |
| Aqua | 64.50 % |
| Parfum | 0.50 % |
| | |

| Phase 2-I | |
|---|---|
| Sodium Laureth Sulfate | 20.00 % |
| Decyl Glucoside | 5.00 % |
| Cocamidopropyl Betaine | 8.00 % |
| Laureth-2 | 2.50 % |
| Polysorbate-20 | 1.00 % |
| Aqua | 63.00 % |
| Parfum | 0.50 % |
| | |

| Phase 2-J | |
|---|---|
| Sodium Myreth Sulfate | 15.00 % |
| Lauryl Glucoside | 10.00 % |
| Laureth-2 | 1.50 % |
| Aqua | 73.00 % |
| Parfum | 0.50 % |
| | |

| Phase 2-K | |
|---|---|
| Emulgade® CM | 20.00 % |
| Polysorbate 20 | 0.80 % |
| Coco-Glucoside | 2.50 % |
| Phenoxyethanol | 1.00 % |
| Cetylpyridinium Cloride | 0.10 % |
| Tetrasodium EDTA | 0.20 % |
| Aqua | 75.22 % |
| Citric Acid | 0.08 % |
| Parfum | 0.10 % |
| | |

| Phase 2-L | |
|---|---|
| Emulgade® SE-PF | 1.66 % |
| Ceteareth-12 | 0.94 % |
| Lamesoft® PO 65 | 0.25 % |
| Paraffinum Liquidum | 3.00 % |
| Cetylpyridinium Cloride | 0.05 % |
| Polysorbate-20 | 1.00 % |
| Citric Acid | 0.03 % |
| Tetrasodium EDTA | 0.20 % |
| Nipaguard® IPF | 0.10 % |
| Aqua | 92.66 % |
| Parfum | 0.11 % |
| | |

| Phase 2-M | |
|---|---|
| Emulgade® SE-PF | 1.627 % |
| Ceteareth-12 | 0.921 % |
| Lamesoft® PO 65 | 0.245 % |
| Paraffinum Liquidum | 2.940 % |
| Glyceryl Polymethacrylate | 2.000 % |
| Cetylpyridinium Cloride | 0.049 % |
| Polysorbate-20 | 0.980 % |
| Citric Acid | 0.029 % |
| Tetrasodium EDTA | 0.196 % |
| Nipaguard® IPF | 0.098 % |
| Aqua | 90.807 % |
| Parfum | 0.108 % |

### Example 3 :

Dry sponge consisting of two parts made of different material are glued together. One part is made of liquid cellulose. After drying, the sponge material forms a layer with the thickness of 37 mm. The sponge has a surface weight of 70 g/m2 and is impregnated with 10 g/m2 of an aqueous phase, which is prepared as set five in example 2. After that the material is cut into blocks of 135 x 90 x 37 mm. The other part of the product is made of polyurethane with the measures 135 x 90 x 5 mm. After gluing both parts together a lipid phase as described in set four of example list 1 was applied with 5 g/article onto the polyurethane side. Subsequently the product was dried with hot air and is wrapped into single packs.

### Example 4 :

Dry sponge made of a mixture of liquid cellulose and a lipid phase, which is prepared as set two in example 1. The sponge has a surface weight of 70 g/m2 and was cut after forming into blocks of 135 x 90 x 37 mm. Lipid addition to the cellulose was set at 5%. Inside the sponge there is a depot of an aqueous lotion according set three of example list. The product is subsequently dried by hot air and wrapped into a single pack.

## Claims

1. A product comprising an applicator other than a porous or absorbent sheet selected from the group consisting of a puff, pad, sponge, cotton ball, swab, brush, glove and mitt, whereto a lipid phase, which has a melting point or melting range of above or equal to 25°C, has been applied in its molten form in a discontinuous pattern, and whereto subsequently an aqueous phase has been applied containing one or more active substances and/or at least one moisturizer, deodorant, skin caring ingredient, plant extract, vitamin, perfume oil, dye, sunscreen filter, hydrotope or self-tanning agent, and which has been dried, wherein the lipid phase comprises
mono-, di- or triglycerides of fatty acids containing from 12 to 24 carbon atoms in an amount of at least 50 % w/w of the total amount of components making up the lipid phase, said glycerides being derived from or present in natural oils; or
wherein the lipid phase contains C₁₂-C₂₄-fatty alcohols; or
wherein the lipid phase contains fatty acids; or
wherein the lipid phase contains dialkyl(ene) ethers or -carbonates, dicarboxylic acids or hydroxy fatty alcohols, or a combination thereof.

2. A product according to claim 1 wherein the lipid phase comprises mono-, di- or triglycerides of fatty acids containing from 16 to 20 carbon atoms.

3. The product according to claim 1 wherein the lipid phase comprises triglycerides selected from glyceryl stearate, glyceryl oleate, glyceryl laurate, glyceryl myristate, cocoglycerides, or hydrogenated palm oil glycerides, hydrogenated castor oil, or hydrogenated rapeseed oil.

4. The product according to any of claims 1, 2 or 3 wherein the lipid phase comprises mono-, di- or triglycerides in an amount of at least 70 %, preferably at least 90 %, w/w of the total amount of components making up the lipid phase.

5. The product according to claim 1 wherein the fatty alcohols are selected from myristyl alcohol, 1-pentadecanol, cetyl alcohol, lauryl alcohol, oleyl alcohol, palmityl alcohol,1-heptadecanol, stearyl alcohol, cetearyl alcohol, 1-nonadecanol, arachidyl alcohol, 1-heneicosanol, behenyl alcohol, brassidyl alcohol, lignoceryl alcohol, ceryl alcohol or myricyl alcohol and C₁₆/C₁₈-Guerbet alcohols.

6. The product according to claim 1 or 5 wherein the fatty alcohols are present in the lipid phase, in an amount relative to the total weight amount of the lipid phase, which is in the range of 1-40%, preferably 1-30 % (w/w), more preferably of 1- 20% (w/w), still more preferably from 1-10 % (w/w).

7. The product according to claim 1 wherein the fatty acids are C₁₄-C₄₀-fatty acids or in particular are C₁₆-C₃₀-fatty acids.

8. The product according to claim 7 wherein the fatty acids are selected from myristic-, pentadecanoic-, palmitic-, margaric-, stearic-, nonadecanoic-, arachic-, behenic-, lignoceric-, cerotic-, melissic-, erucaic-, elaeostearic-, oleic-, linoleic-, lauric acid and hydroxy-substituted fatty acids.

9. The product according to claim 1 or 8 wherein the total amount of the fatty acids present in the lipid phase, relative to the total weight amount of the lipid phase, is in the range of 1-30 % (w/w), preferably of 1-20% (w/w), more preferably from 1- 10 % (w/w).

10. The product according to claim 1 wherein the lipid phase contains one or more of components (a), (b), (c), (d), (e) or(f) as defined hereafter:
(a) at least 1-50 % (w/w), in particular at least 1-10% of an oily or waxy component
(b) 0,1-5 % (w/w) of at least one active ingredient
(c) 1-10 % (w/w) of at least one oil
(d) 0.1-10 % (w/w) of at least one emulsifier
(e) 5-90 % (w/w) of further waxy components (f) 0-5 % (w/w) water.

11. The product according to claim 10 wherein the lipid phase contains all components (a)-(f).

12. The product according to claim 10 or 11 wherein component (a) is an oily or waxy component selected from C₁₄-C₃₀-dialkyl ethers, C₁₄-C₃₀-dialkyl carbonates, C₄-C₃₄-dicarbonic acids or C₁₂-C₃₀-hydroxy fatty alcohols or mixtures thereof.

13. The product according to any of claims 1 to 12 wherein the lipid phase contains one or more active substances.

14. The product according to any of claims 1 or 13 wherein the active substance(s) is or are anti-microbials, e.g. anti-bacterials and antifungals, anti-inflammatory agents, anti-irritating, anti-itching, anti-perspirant agents.

15. The product according to any of claims 1 to 12 wherein the lipid phase contains at least one moisturizer, deodorant, skin caring ingredient, plant extract, vitamin, perfume oil, dye, sunscreen filter, hydrotope or self-tanning agent.

16. The product according to any of claims 1 to 12, wherein the lipid or the aqueous phase contains at least one emulsifier.

17. The product according to any of claims 1 to 12 wherein the lipid phase contains at least one superfatting agent, thickener, cationic polymer, anionic polymer, zwitterionic polymer, amphoteric polymer, consistency agent, anti-oxidant.

18. The product according to any of claims 1 to 12 wherein the lipid or the aqueous phase contains an insect repellent, a sunscreen filter, a powder or a peeling agent.

19. A method of manufacturing a product as claimed in any of claims 1 to 18, said method comprising contacting the surface of the applicator with a lipid phase in its molten form in a discontinuous pattern, and subsequently applying an aqueous phase comprising one or more active substances and/or at least one moisturizer, deodorant, skin caring ingredient, plant extract, vitamin, perfume oil, dye, sunscreen filter, hydrotope or self-tanning agent, and wherein the drying step takes place after the aqueous phase has been applied.

20. The method according to claim 19, wherein the aqueous phase is applied by spraying, drippling, immersing or running through a bath, and the lipid phase is applied by spraying, contacting, printing or a direct contact process where there is a direct contact between the applicator and an application head having slit nozzles.

21. The method according to claim 19 or 20 wherein the drying step comprises the application of hot air, or by leading the wet sheet through an oven or over an heated or warmed transport roll.

22. Use of a product as claimed in any of claims 1 to 18 as a combined cleanser and applicator of active substances.

## Patentansprüche

1. Produkt, umfassend einen Applikator, der kein poröses oder absorbierendes Tuch ist, ausgewählt aus der Gruppe bestehend aus einem Bausch, einem Kissen, einem Schwamm, einem Watteball, einem Tupfer, einer Bürste, einem Fingerhandschuh und einem Fausthandschuh, auf den eine flüssige Phase, die einen Schmelzpunkt oder Schmelzbereich von über oder gleich 25 °C hat, in ihrer geschmolzenen Form in einem diskontinuierlichen Muster aufgebracht wurde und auf den anschließend eine wässrige Phase aufgebracht wurde, die eine oder mehrere Wirkstoffe und/oder zumindest ein Feuchtigkeitsmittel, ein Deodorant, einen Hautpflegebestandteil, einen Pflanzenextrakt, ein Vitamin, ein Duftöl, einen Farbstoff, einen Sonnenschutzfilter, eine hydrotrope Verbindung oder ein Selbstbräunungsmittel enthält, und der getrocknet wurde, wobei die Lipidphase
Mono-, Di- oder Triglyceride von Fettsäuren, die 12 bis 24 Kohlenstoffatome enthalten, in einer Menge von zumindest 50 Gew.-% der Gesamtmenge der Komponenten umfasst, aus denen sich die Lipidphase zusammensetzt, wobei die Glyceride aus natürlichen Ölen gewonnen werden oder in diesen vorliegen, oder
wobei die Lipidphase C₁₂-C₂₄-Fettalkohole enthält, oder
wobei die Lipidphase Fettsäuren enthält, oder
wobei die Lipidphase Dialkyl(en)ether oder -carbonate, Dicarbonsäuren oder Hydroxyfettalkohole oder eine Kombination daraus enthält.

2. Produkt nach Anspruch 1, **dadurch gekennzeichnet, dass** die Lipidphase Mono-, Di- oder Triglyceride von Fettsäuren umfasst, die 16 bis 20 Kohlenstoffatome enthalten.

3. Produkt nach Anspruch 1, **dadurch gekennzeichnet, dass** die Lipidphase Triglyceride umfasst, ausgewählt aus Glycerylstearat, Glyceryloleat, Glyceryllaurat, Glycerylmyristat, Kokosglyceriden oder hydrierten Palmölglyceriden, hydriertem Rizinusöl oder hydriertem Rapsöl.

4. Produkt nach einem der Ansprüche 1, 2 oder 3, **dadurch gekennzeichnet, dass** die Lipidphase Mono-, Di- oder Triglyceride in einer Menge von zumindest 70 Gew.-%, bevorzugt zumindest 90 Gew.-% der Gesamtmenge der Komponenten, aus denen sich die Lipidphase zusammensetzt, umfasst.

5. Produkt nach Anspruch 1, **dadurch gekennzeichnet, dass** die Fettalkohole ausgewählt sind aus Myristylalkohol, 1-Pentadecanol, Cetylalkohol, Laurylalkohol, Oleylalkohol, Palmitylalkohol, 1-Heptadecanol, Stearylalkohol, Cetearylalkohol, 1-Nonadecanol, Arachidylalkohol, 1-Heneicosanol, Behenylalkohol, Brassidylalkohol, Lignocerylalkohol, Cerylalkohol oder Myricylalkohol und C₁₆/C₁₈-Guerbet-Alkoholen.

6. Produkt nach Anspruch 1 oder 5, **dadurch gekennzeichnet, dass** die Fettalkohole in der Lipidphase in einer Menge bezogen auf die Gesamtgewichtsmenge der Lipidphase vorliegen, die im Bereich von 1 bis 40 Gew.%, bevorzugt 1 bis 30 Gew.-%, bevorzugter 1 bis 20 Gew.-% und noch bevorzugter 1 bis 10 Gew.-% liegt.

7. Produkt nach Anspruch 1, **dadurch gekennzeichnet, dass** die Fettsäuren C₁₄-C₄₀-Fettsäuren oder insbesondere C₁₆-C₃₀-Fettsäuren sind.

8. Produkt nach Anspruch 7, **dadurch gekennzeichnet, dass** die Fettsäuren ausgewählt sind aus Myristin-, Pentadecan-, Palmitin-, Margarin-, Stearin-, Nonadecan-, Arachin-, Behenin-, Lignocerin-, Xerotin-, Melissin-, Eruca-, Elaeostearin-, Öl-, Linol-, Laurinsäure und hydroxysubstituierten Fettsäuren.

9. Produkt nach Anspruch 1 oder 8, **dadurch gekennzeichnet, dass** die Gesamtmenge der in der Lipidphase vorliegenden Fettsäuren bezogen auf die Gesamtgewichtsmenge der Lipidphase im Bereich von 1 bis 30 Gew.-%, bevorzugt 1 bis 20 Gew.-%, bevorzugter 1 bis 10 Gew.-% liegt.

10. Produkt nach Anspruch 1, **dadurch gekennzeichnet, dass** die Lipidphase eine oder mehrere der im Folgenden definierten Komponenten (a), (b), (c), (d), (e) oder (f) enthält:
(a) zumindest 1 bis 50 Gew.-%, insbesondere zumindest 1 bis 10 Gew.-%, einer ölartigen oder wachsartigen Komponente
(b) 0,1 bis 5 Gew.-% zumindest eines Wirkstoffs
(c) 1 bis 10 Gew.-% zumindest eines Öls
(d) 0,1 bis 10 Gew.-% zumindest eines Emulgators
(e) 5 bis 90 Gew.-% weiterer wachsartiger Komponenten
(f) 0 bis 5 Gew.-% Wasser.

11. Produkt nach Anspruch 10, **dadurch gekennzeichnet, dass** die Lipidphase alle Komponenten (a) bis (f) enthält.

12. Produkt nach Anspruch 10 oder 11, **dadurch gekennzeichnet, dass** Komponente (a) eine ölartige oder wachsartige Komponente ist, ausgewählt aus C₁₄-C₃₀-Dialkylethern, C₁₄-C₃₀-Dialkylcarbonaten, C₄-C₃₄-Dicarbonsäuren oder C₁₂-C₃₀-Hydroxyfettalkoholen oder Gemischen daraus.

13. Produkt nach einem der Ansprüche 1 bis 12, **dadurch gekennzeichnet, dass** die Lipidphase einen oder mehrere Wirkstoffe enthält.

14. Produkt nach einem der Ansprüche 1 oder 13, **dadurch gekennzeichnet, dass** der Wirkstoff (die Wirkstoffe) antimikrobielle Wirkstoffe, zum Beispiel antibakterielle und antifungale Wirkstoffe, entzündungshemmende Wirkstoffe, reizlindernde, juckreizstillende, schweißhemmende Wirkstoffe ist oder sind.

15. Produkt nach einem der Ansprüche 1 bis 12, **dadurch gekennzeichnet, dass** die Lipidphase zumindest ein Feuchtigkeitsmittel, ein Deodorant, einen Hautpflegebestandteil, einen Pflanzenextrakt, ein Vitamin, ein Duftöl, einen Farbstoff, einen Sonnenschutzfilter, eine hydrotrope Verbindung oder ein Selbstbräunungsmittel enthält.

16. Produkt nach einem der Ansprüche 1 bis 12, **dadurch gekennzeichnet, dass** die Lipid- oder die wässrige Phase zumindest einen Emulgator enthält.

17. Produkt nach einem der Ansprüche 1 bis 12, **dadurch gekennzeichnet, dass** die Lipidphase zumindest ein superfettendes Mittel, Verdickungsmittel, kationisches Polymer, anionisches Polymer, zwitterionisches Polymer, amphoteres Polymer, Konsistenzmittel, Antioxidationsmittel enthält.

18. Produkt nach einem der Ansprüche 1 bis 12, **dadurch gekennzeichnet, dass** die Lipid- oder die wässrige Phase ein Insektenschutzmittel, einen Sonnenschutzfilter, einen Puder oder ein Peelingmittel enthält.

19. Verfahren zur Herstellung eines Produkts nach einem der Ansprüche 1 bis 18, wobei das Verfahren das Inkontaktbringen der Oberfläche des Applikators mit einer Lipidphase in ihrer geschmolzenen Form in einem diskontinuierlichen Muster und das anschließende Aufbringen einer wässrigen Phase, umfassend einen oder mehrere Wirkstoffe und/oder zumindest ein Feuchtigkeitsmittel, ein Deodorant, einen Hautpflegebestandteil, einen Pflanzenextrakt, ein Vitamin, ein Duftöl, einen Farbstoff, einen Sonnenschutzfilter, eine hydrotrope Verbindung oder ein Selbstbräunungsmittel, umfasst und wobei der Trocknungsschritt erfolgt, nachdem die wässrige Phase aufgebracht wurde.

20. Das Verfahren nach Anspruch 19, **dadurch gekennzeichnet, dass** die wässrige Phase durch Sprühen, Tröpfeln, Eintauchen oder Ziehen durch ein Bad aufgebracht wird und die Lipidphase durch Sprühen, Inkontaktbringen, Drucken oder einen direkten Kontaktprozess, bei dem ein direkter Kontakt zwischen dem Applikator und einem Applikationskopf mit Schlitzdüsen vorhanden ist, aufgebracht wird.

21. Verfahren nach Anspruch 19 oder 20, **dadurch gekennzeichnet, dass** der Trocknungsschritt die Anwendung von Heißluft oder das Führen des nassen Tuchs durch einen Ofen oder über eine erhitzte oder erwärmte Transportrolle umfasst.

22. Verwendung eines Produkts nach einem der Ansprüche 1 bis 18 als kombinierten Reiniger und Applikator von Wirkstoffen.

## Revendications

1. Produit comprenant un applicateur, autre qu'une feuille absorbante ou poreuse, sélectionné à partir du groupe constitué d'une houppette, une compresse, une éponge, un tampon d'ouate, un coton-tige, un pinceau, un gant, et une mitaine, sur lequel une phase liquide, ayant un point de fusion ou une plage de fusion égal(e) ou supérieur(e) à 25°C, a été appliquée dans sa forme fondue selon un motif discontinu, et sur lequel a été ultérieurement appliquée une phase aqueuse contenant une ou plusieurs substances actives et/ou au moins une crème hydratante, un déodorant, un ingrédient de soin de la peau, un extrait de plante, une vitamine, une huile parfumée, une matière colorante, un filtre solaire, un agent hydrotrope ou autobronzant, et qui a été séché, dans lequel la phase liquide comprend
des monoglycérides, diglycérides et triglycérides d'acides gras contenant de 12 à 24 atomes de carbone en une quantité d'au moins 50 % en poids/poids de la quantité totale de composants pour préparer la phase liquide, lesdits glycérides étant dérivés à partir de ou présents dans des huiles naturelles, ou
dans lequel la phase liquide contient des alcools gras en C₁₂-C₂₄ ; ou
dans lequel la phase liquide contient des acides gras ; ou
dans lequel la phase liquide contient des éthers de dialkyl(ène) ou des carbonates de dialkyl(ène), des acides dicarboxyliques ou des alcools gras hydroxy, ou une combinaison de ceux-ci.

2. Produit selon la revendication 1, dans lequel la phase liquide comprend des monoglycérides, diglycérides ou triglycérides d'acides gras contenant de 16 à 20 atomes de carbone.

3. Produit selon la revendication 1, dans lequel la phase liquide comprend des triglycérides sélectionnés parmi du stéarate de glycéryle, de l'oléate de glycéryle, du laurate de glycéryle, du myristate de glycéryle, des cocoglycérides, ou des glycérides d'huile de palme hydrogénée, de l'huile de ricin hydrogénée, ou de l'huile de colza hydrogénée.

4. Produit selon l'une quelconque des revendications 1, 2 ou 3, dans lequel la phase liquide comprend des monoglycérides, diglycérides ou triglycérides en une quantité d'au moins 70 %, de manière préférée d'au moins 90 %, en poids/poids de la quantité totale des composants pour préparer la phase liquide.

5. Produit selon la revendication 1, dans lequel les alcools gras sont sélectionnés parmi de l'alcool myristique, du 1-pentadécanol, de l'alcool cétylique, de l'alcool laurylique, de l'alcool oléylique, de l'alcool palmitique, du 1-heptadécanol, de l'alcool stéarylique, de l'alcool cétéarylique, du 1-nonadécanol, de l'alcool arachidylique, du 1-hénéicosanol, de l'alcool béhénylique, de l'alcool brassidylique, de l'alcool lignocérylique, de l'alcool cérylique, ou de l'alcool myricylique, et des alcools de Guerbet en C₁₆-C₁₈.

6. Produit selon la revendication 1 ou 5, dans lequel les alcools gras sont présents dans la phase liquide, en une quantité par rapport au poids total de la phase liquide qui est dans la plage de 1 à 40 %, de manière préférée de 1 à 30 % (poids/poids), de manière plus préférée de 1 à 20 % (poids/poids), de manière encore plus préférée de 1 à 10 % (poids/poids).

7. Produit selon la revendication 1, dans lequel les acides gras sont des acides gras en C₁₄-C₄₀ ou sont en particulier des acides gras en C₁₆-C₃₀.

8. Produit selon la revendication 7, dans lequel les acides gras sont sélectionnés parmi de l'acide myristique, pentadécanoïque, palmitique, margarique, stéarique, nonadécanoïque, arachidique, béhénique, lignocérique, cérotique, mélissique, érucique, éléostéarique, oléique, linoléique, laurique et des acides gras substitués par de l'hydroxy.

9. Produit selon la revendication 1 ou 8, dans lequel la quantité totale des acides gras présents dans la phase liquide, par rapport à la quantité totale en poids de la phase liquide, est dans la plage de 1 à 30 % (poids/poids), de manière préférée de 1 à 20 % (poids/poids), de manière plus préférée de 1 à 10 % (poids/poids).

10. Produit selon la revendication 1, dans lequel la phase liquide contient un ou plusieurs composants (a), (b), (c), (d), (e), ou (f) tels que définis ci-dessus :
(a) au moins 1 à 50 % (poids/poids), en particulier au moins 1 à 10 % d'un composant huileux ou cireux,
(b) 0,1 à 5 % (poids/poids) d'au moins un ingrédient actif,
(c) 1 à 10 % (poids/poids) d'au moins une huile,
(d) 0,1 à 10 % (poids/poids) d'au moins un émulsifiant,
(e) 5 à 90 % (poids/poids) de composants cireux additionnels, et (f) 0 à 5 % (poids/poids) d'eau.

11. Produit selon la revendication 10, dans lequel la phase liquide contient tous les composants (a) à (f).

12. Produit selon la revendication 10 ou 11, dans lequel le composant (a) est un composant huileux ou cireux sélectionné parmi des éthers de dialkyle en C₁₄₋₃₀, des carbonates de dialkyle en C₁₄₋₃₀, des acides dicarboxyliques en C₄₋₃₄ ou des alcools gras d'hydroxy en C₁₂-C₃₀ ou des mélanges de ceux-ci.

13. Produit selon l'une quelconque des revendications 1 à 12, dans lequel la phase liquide contient une ou plusieurs substances actives

14. Produit selon l'une quelconque des revendications 1 ou 13, dans lequel la ou les substance(s) active(s) est ou sont antimicrobienne(s), par exemple des substances antibactériennes et des substances antifongiques, des agents anti-inflammatoire(s), des agents anti-irritants, des agents antihistaminiques, des agents antitranspirants.

15. Produit selon l'une quelconque des revendications 1 à 12, dans lequel la phase liquide contient au moins une crème hydratante, un déodorant, un ingrédient de soin de la peau, un extrait de plante, une vitamine, une huile parfumée, une matière colorante, un filtre solaire, un agent hydrotrope ou autobronzant.

16. Produit selon l'une quelconque des revendications 1 à 12, dans lequel la phase liquide ou la phase aqueuse contient au moins un émulsifiant.

17. Produit selon l'une quelconque des revendications 1 à 12, dans lequel la phase liquide contient au moins un agent surgraissant, un épaississant, un polymère cationique, un polymère anionique, un polymère zwitterionique, un polymère amphotère, un agent de consistance, un antioxydant.

18. Produit selon l'une quelconque des revendications 1 à 12, dans lequel la phase liquide ou la phase aqueuse contient un insectifuge, un filtre solaire, une poudre ou un agent de pelage.

19. Procédé de fabrication d'un produit selon l'une quelconque des revendications 1 à 18, ledit procédé comprenant la mise en contact de la surface de l'applicateur avec une phase liquide dans sa forme fondue selon un motif discontinu, et l'application ultérieure d'une phase aqueuse comprenant une ou plusieurs substances actives et/ou au moins une crème hydratante, un déodorant, un ingrédient de soin de la peau, un extrait de plante, une vitamine, une huile parfumée, une matière colorante, un filtre solaire, un agent hydrotrope ou autobronzant, et dans lequel l'étape de séchage lieu après que la phase aqueuse a été appliquée.

20. Procédé selon la revendication 19, dans lequel la phase aqueuse est appliquée par pulvérisation, goutte à goutte, immersion ou passage à travers un bain, et la phase liquide est appliquée par pulvérisation, mise en contact, impression ou un procédé de contact direct dans lequel il y a un contact direct entre l'applicateur et une tête d'application présentant des buses à fente.

21. Procédé selon la revendication 19 ou 20, dans lequel l'étape de séchage comprend l'application d'air chaud, ou a lieu en guidant la feuille humide à travers un four ou au-dessus d'un rouleau de transport chauffé ou réchauffé.

22. Utilisation d'un produit selon l'une quelconque des revendications 1 à 18 en tant que combinaison d'un nettoyant et d'un applicateur de substances actives.
